(19) **Europäisches Patentamt**
**European Patent Office**
**Office européen des brevets**

(11) **EP 4 173 565 B1**

(12) **EUROPEAN PATENT SPECIFICATION**

(45) Date of publication and mention
of the grant of the patent:
**23.08.2023 Bulletin 2023/34**

(21) Application number: **22179861.4**

(22) Date of filing: **20.06.2022**

(51) International Patent Classification (IPC):
*A61B 5/11* *(2006.01)* *A61B 5/00* *(2006.01)*

(52) Cooperative Patent Classification (CPC):
**A61B 5/6829; A61B 5/1116; A61B 5/112;**
**A61B 5/1123; A61B 5/7264;** A61B 2562/0219

(54) **INFORMATION PROCESSING PROGRAM, INFORMATION PROCESSING METHOD, AND INFORMATION PROCESSING DEVICE**

INFORMATIONSVERARBEITUNGSPROGRAMM,
INFORMATIONSVERARBEITUNGSVERFAHREN UND
INFORMATIONSVERARBEITUNGSVORRICHTUNG

PROGRAMME, PROCÉDÉ ET DISPOSITIF DE TRAITEMENT D'INFORMATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2021 JP 2021174949**

(43) Date of publication of application:
**03.05.2023 Bulletin 2023/18**

(73) Proprietor: **FUJITSU LIMITED**
**Kawasaki-shi, Kanagawa 211-8588 (JP)**

(72) Inventors:
• **Kawasaki, Kunimasa**
**Kawasaki-shi, Kanagawa, 211-8588 (JP)**

• **Kusumine, Naruhiro**
**Kawasaki-shi, Kanagawa, 211-8588 (JP)**
• **Hotta, Shinji**
**Kawasaki-shi, Kanagawa, 211-8588 (JP)**
• **Otsuka, Hiroshi**
**Kawasaki-shi, Kanagawa, 211-8588 (JP)**
• **Inomata, Akihiro**
**Kawasaki-shi, Kanagawa, 211-8588 (JP)**

(74) Representative: **Hoffmann Eitle**
**Patent- und Rechtsanwälte PartmbB**
**Arabellastraße 30**
**81925 München (DE)**

(56) References cited:
**EP-A1- 3 847 961**

**Description**

FIELD

The embodiments discussed herein are related to an information processing program, an information processing method, and an information processing device.

BACKGROUND

In a medical field, it has been expected in the past to grasp a health condition of a patient in order to provide appropriate medical treatment to the patient. For example, there is a case where an attempt is made to grasp the health condition of the patient during a time span when the patient walks normally, on the basis of measured values obtained from a sensor device attached to the patient. The normal walking refers to, for example, traveling by walking in a natural posture in a straight-going manner.

As the prior art, for example, there is one that gives meanings to walking sections in time-series data and gives a meaning to a direction change section. In addition, for example, there is a technique that detects a movement of a patient from sensor data and classifies the movement of the patient. In addition, for example, there is a technique that computes the walking cycle of walking and extracts walking in a state in which the walking cycle is stable. In addition, for example, there is a technique that extracts a portion where a lower limb rotation motion with the direction of gravity as an axis occurs, as a direction change motion candidate.

International Publication Pamphlet No. WO 2018/042525, U.S. Patent Application Publication No. 2017/0281054, International Publication Pamphlet No. WO 2020/045371, European patent application publication No. EP 3 847 961 A1, and International Publication Pamphlet No. WO 2017/109920 are disclosed as related art.

SUMMARY

There is provided an information processing program as set out in Claim 1. There is also provided an information processing method as set out in Claim 7. There is also provided an information processing device as set out in Claim 8.

BRIEF DESCRIPTION OF DRAWINGS

FIG. 1 is an explanatory diagram illustrating an example of an information processing method according to an embodiment;
FIG. 2 is an explanatory diagram illustrating an example of an information processing system 200;
FIG. 3 is a block diagram illustrating a hardware configuration example of an information processing device 100;
FIG. 4 is a block diagram illustrating a hardware configuration example of an information measuring device 201;
FIG. 5 is a block diagram illustrating a functional configuration example of the information processing device 100;
FIG. 6 is a block diagram illustrating a specific functional configuration example of the information processing device 100;
FIG. 7 is an explanatory diagram illustrating an example of classification of a plurality of sections;
FIG. 8 is an explanatory diagram (part 1) illustrating an example of calculating a base index value;
FIG. 9 is an explanatory diagram (part 2) illustrating an example of calculating a base index value;
FIG. 10 is an explanatory diagram (part 3) illustrating an example of calculating a base index value;
FIG. 11 is an explanatory diagram (part 4) illustrating an example of calculating a base index value;
FIG. 12 is an explanatory diagram (part 1) illustrating an example of calculating an index value of each stride section;
FIG. 13 is an explanatory diagram (part 2) illustrating an example of calculating an index value of each stride section;
FIG. 14 is an explanatory diagram (part 3) illustrating an example of calculating an index value of each stride section;
FIG. 15 is an explanatory diagram (part 1) illustrating an example of extracting a normal walking section;
FIG. 16 is an explanatory diagram (part 2) illustrating an example of extracting a normal walking section;
FIG. 17 is a flowchart illustrating an example of a setting processing procedure; and
FIG. 18 is a flowchart illustrating an example of a determination processing procedure.

DESCRIPTION OF EMBODIMENTS

[TECHNICAL PROBLEM]

However, with the past technique, it is difficult to acquire the measured values during a time span when the patient walks normally. For example, it is difficult to identify the time span during which the patient walks normally, in the period in which the patient walks at the patient's home, outdoors, or the like without the supervision of a healthcare professional. Accordingly, it is difficult to extract the measured values during a time span when the patient walks normally, from among the measured values obtained from a sensor device attached to the patient.

In one aspect, the present embodiments aim to identify a section in which a subject has traveled in a predetermined walking manner.

[SOLUTION TO PROBLEM]

According to an aspect of the embodiments, an information processing program that causes a computer to execute a process includes acquiring first time-series data that includes measured values regarding movement of legs of a subject in a first period in which the subject travels in a walking manner for each of a plurality of axial directions; acquiring a first index value that relates to value of the measured values of one axial direction of the plurality of axial directions over value of the measured values of another axial direction of the plurality of axial directions for a first section in which the subject swings one of the legs for each of first stride sections that corresponds to one step of the subject in the first period based on the acquired first time-series data; acquiring second time-series data that includes second measured values regarding the movement of the legs of the subject in a second period different from the first period in which the subject travels in the walking manner for each of the plurality of axial directions; acquiring a second index value that relates to value of the measured values of one axial direction of the plurality of axial directions over value of the measured values of another axial direction of the plurality of axial directions for a second section in which the subject swings one of the legs for each of second stride sections that corresponds to one step of the subject in the second period based on the acquired second time-series data; and determining whether or not each of the second stride sections in the second period corresponds to the walking manner, based on the acquired first index value and the acquired second index value.

[ADVANTAGEOUS EFFECTS OF INVENTION]

According to one mode, it may become possible to identify a section in which the subject has traveled in a predetermined walking manner.

Hereinafter, embodiments of an information processing program, an information processing method, and an information processing device will be described in detail with reference to the drawings.

(One Example of Information Processing Method According to Embodiment)

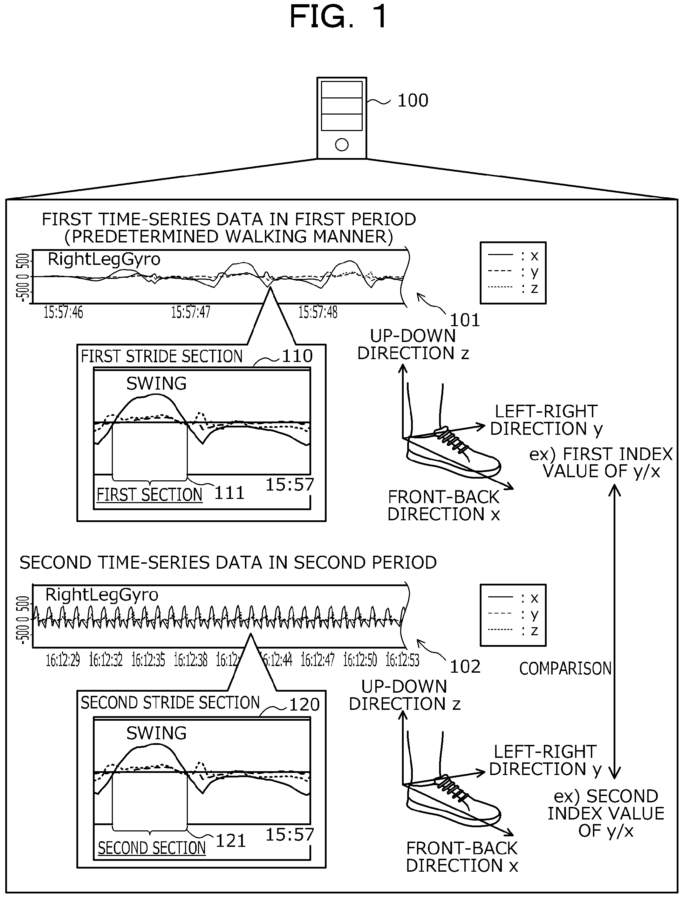

FIG. 1 is an explanatory diagram illustrating one example of an information processing method according to an embodiment. An information processing device 100 is a computer for identifying a section in which a subject has traveled in a predetermined walking manner. The predetermined walking manner is, for example, a walking manner in normal walking. Walking is, for example, a state in which the subject travels using own legs of the subject.

In a medical field, it has been expected in the past to grasp a health condition of a patient in order to provide appropriate medical treatment to the patient. For example, there is a case where a 10 m walking test is conducted under the supervision of a healthcare professional, and an attempt is made to grasp the health condition of the patient for a time span during which the patient walks normally with a sensor device worn, on the basis of measured values obtained from the sensor device attached to the patient.

In regard to this, if the daily health condition of the patient can be grasped for a time span during which the patient is not under the supervision of a healthcare professional, it is considered to be possible to more efficiently provide appropriate medical treatment to the patient. For example, in the area of long-term care, the formulation of a guidance plan may be facilitated on the basis of the daily health condition of the patient.

However, with the past technique, it is difficult to acquire the measured values during a time span when the patient walks normally. For example, it is difficult to identify the time span during which the patient walks normally, in the period in which the patient walks at the patient's home, outdoors, or the like without the supervision of a healthcare professional. Accordingly, it is difficult to extract the measured values during the time span when the patient walks normally, from among the measured values obtained from the sensor device attached to the patient, and it may not be possible to grasp the daily health condition of the patient, on the basis of the measured values during the time span when the patient walks normally.

For example, when the patient walks at the patient's home, outdoors, or the like, there is a case where acceleration, deceleration, direction change, avoidance motion, or the like is performed, and it is difficult to identify the time span during which the patient walks normally, in the period in which the patient walks at the patient's home, outdoors, or the like. Accordingly, it is difficult to extract the measured values during the time span when the patient walks normally, from among the measured values obtained from the sensor device attached to the patient, and it may not be possible to grasp the daily health condition of the patient, on the basis of the measured values during the time span when the patient walks normally.

For example, an approach intended to identify the time span during which the patient walks linearly, using a global positioning system (GPS) receiver attached to the patient and a pedometer attached to the patient is conceivable. This approach has a disadvantage that, when the patient walks at the patient's home, the strength of the GPS signal to be received by the GPS receiver is low, and it is difficult to identify the time span during which the patient walks linearly. For this approach, for example, the above-mentioned International Publication Pamphlet No. WO 2020/045371 can be referred to.

For example, an approach intended to identify the time span during which the patient changed the direction, using the values of motion sensors attached to the left and right legs of the patient and identify the time span during which the patient walks linearly is conceivable. This approach is not allowed to take motions other than a relatively large direction change into account and has a disadvantage that it is difficult to identify the time span during which the patient walks linearly. For this approach, for example, the above-mentioned International Publication Pamphlet No. WO 2017/109920 can be referred to.

In addition, there is a case the patient does not walk normally even if a 10 m walking test is conducted under the supervision of a healthcare professional. For example, due to tension at a medical institution or the like, the patient sometimes does not walk normally instead of going straight in a natural posture. For this reason, it is difficult to acquire the measured values during the time span when the patient walks normally. Accordingly, even if the patient is under the supervision of a healthcare professional, it is desired in some cases to extract the measured values during the time span when the patient walks normally, from among the measured values obtained from the sensor device attached to the patient.

Thus, in the present embodiment, an information processing method capable of identifying a section in which the subject has traveled in a predetermined walking manner will be described.

In FIG. 1, (1-1) the information processing device 100 acquires first time-series data 101. The first time-series data 101 includes measured values regarding the movement of the legs of the subject in a first period in each axial direction among a plurality of axial directions. In the example in FIG. 1, for example, the first time-series data 101 includes measured values regarding the movement of the right leg of the subject in the first period in each axial direction among a plurality of axial directions.

The first period is, for example, a period in which the subject is deemed to have traveled in a predetermined walking manner. For example, the first period is a period in which the subject is deemed to have walked normally. The plurality of axial directions includes, for example, a front-back direction, a left-right direction, and an up-down direction of the legs of the subject. The front-back direction is, for example, an x-axis direction. The left-right direction is, for example, a y-axis direction. The up-down direction is, for example, a z-axis direction. The information processing device 100 acquires the first time-series data 101 by accepting the input of the first time-series data 101, for example, on the basis of an operation input from a user.

(1-2) On the basis of the acquired first time-series data 101, the information processing device 100 calculates a first index value in the first period for a first section 111 in which the subject swings one leg, for each first stride section 110 corresponding to one step of the subject. The first index value is, for example, an index value relating to a measured value in one axial direction based on a measured value in another axial direction,

among the plurality of axial directions.

For example, the first index value is the ratio of a statistical value of the measured values in the y-axis direction to a statistical value of the measured values in the x-axis direction. For example, the statistical value is an average value, a median, a mode, a maximum value, a minimum value, or the like. For example, on the basis of the first time-series data 101, the information processing device 100 calculates the ratio of the statistical value of the measured values in the y-axis direction to the statistical value of the measured values in the x-axis direction in the first period for the first section 111 for each first stride section 110. The information processing device 100 sets, for example, the calculated ratio as the first index value.

(1-3) The information processing device 100 acquires second time-series data 102. The second time-series data 102 includes measured values regarding the movement of the legs of the subject in a second period, in each axial direction. In the example in FIG. 1, for example, the second time-series data 102 includes measured values regarding the movement of the right leg of the subject in the second period, in each axial direction.

The second period is a period different from the first period. The second period is, for example, a period in which the subject is deemed to have walked in a daily way. The daily walking is, for example, traveling by walking in a daily manner. The plurality of axial directions includes, for example, a front-back direction, a left-right direction, and an up-down direction of the legs of the subject. The front-back direction is, for example, the x-axis direction. The left-right direction is, for example, the y-axis direction. The up-down direction is, for example, the z-axis direction. The information processing device 100 acquires the second time-series data 102 by accepting the input of the second time-series data 102, for example, on the basis of an operation input from a user.

(1-4) On the basis of the acquired second time-series data 102, the information processing device 100 calculates a second index value in the second period for a second section 121 in which the subject swings one leg, for each second stride section 120 corresponding to one step of the subject. The second index value is, for example, an index value relating to a measured value in one axial direction based on a measured value in another axial direction, among the plurality of axial directions.

For example, the second index value is the ratio of a statistical value of the measured values in the y-axis direction to a statistical value of the measured values in the x-axis direction. For example, the statistical value is an average value, a median, a mode, a maximum value, a minimum value, or the like. For example, on the basis of the second time-series data 102, the information processing device 100 calculates the ratio of the statistical value of the measured values in the y-axis direction to the statistical value of the measured values in the x-axis direction in the second period for the second section 121 for each second stride section 120. The information processing device 100 sets, for example, the calculated ratio as the second index value.

(1-5) The information processing device 100 determines whether or not each of the second stride sections 120 in the second period corresponds to the predetermined walking manner, on the basis of the calculated first index value and the calculated second index value. For example, when the difference between the calculated first index value and the second index value calculated for any one of the second stride sections 120 is relatively small, the information processing device 100 determines that the any one of the second stride sections 120 corresponds to the normal walking. The normal walking refers to, for example, traveling by walking in a natural posture in a straight-going manner. For example, when the percentage of the second index value calculated for any one of the second stride sections 120 to the calculated first index value is equal to or lower than a predetermined threshold value, the information processing device 100 determines that the any one of the second stride sections 120 corresponds to the normal walking.

On the other hand, for example, when the difference between the calculated first index value and the second index value calculated for any one of the second stride sections 120 is relatively large, the information processing device 100 determines that the any one of the second stride sections 120 does not correspond to the normal walking. For example, when the percentage of the second index value calculated for any one of the second stride sections 120 to the calculated first index value exceeds a predetermined threshold value, the information processing device 100 determines that the any one of the second stride sections 120 does not correspond to the normal walking.

This allows the information processing device 100 to identify the second stride section 120 in the second period in which the subject has traveled in the predetermined walking manner. The information processing device 100 may identify, for example, the second stride section 120 in the second period in which the subject walked normally. Therefore, the information processing device 100 may identify, for example, the measured values in each axial direction among the plurality of axial directions in the second stride section 120 in which the subject walked normally, which is useful information when the health condition of the subject is analyzed.

As a result, the information processing device 100 may facilitate the analysis of the health condition of the subject, for example. In addition, for example, the information processing device 100 may suppress erroneously analyzing the health condition of the subject on the basis of the measured values in each axial direction among the plurality of axial directions in a section in which the subject did not walk normally.

For example, even if the subject walks at the subject's home, outdoors, or the like in the second period without the supervision of a healthcare professional, the information processing device 100 is allowed to identify the second stride section 120 in the second period in which the subject walked normally. Therefore, for example, the information processing device 100 may facilitate the analysis of the daily health condition of the subject who is not under the supervision of a healthcare professional. In addition, for example, the information processing device 100 may reduce the workload imposed on the healthcare professional.

For example, even if the subject has performed acceleration, deceleration, direction change, avoidance motion, or the like in the second period, the information processing device 100 may identify the second stride section 120 in the second period in which the subject walked normally. Therefore, for example, the information processing device 100 may facilitate the analysis of the daily health condition of the subject who is not under the supervision of a healthcare professional. In addition, for example, the information processing device 100 may reduce the workload imposed on the healthcare professional. Furthermore, for example, the information processing device 100 may allow the subject to not be careful about the walking manner in the second period and may improve the convenience.

For example, even if the subject walks indoors such as the subject's home in the second period, the information processing device 100 may identify the second stride section 120 in the second period in which the subject walked normally. Therefore, for example, the information processing device 100 may facilitate the analysis of the daily health condition of the subject who is not under the supervision of a healthcare professional. In addition, for example, the information processing device 100 may reduce the workload imposed on the healthcare professional.

For example, the information processing device 100 may allow the subject to not be careful about the walking manner in the second period even if the subject is under the supervision of a healthcare professional and may reduce the workload imposed on the healthcare professional, which may improve the convenience.

Here, the case where the predetermined walking manner is the walking manner in the normal walking has been described, but the predetermined walking manner is not limited to this. For example, the predetermined walking manner may be a special walking manner that is not the normal walking. The special walking manner may be, for example, a walking manner when the subject travels on stairs, an inclined surface, or the like. In addition, the special walking manner may be, for example, a walking manner at an abnormal time such as when the subject is injured. In addition, the special walking manner may be, for example, a walking manner having an adverse effect on health, such as when the subject travels with a stoop. In addition, the special walking manner may be, for example, a walking manner having a risk of an accident, such as when the subject travels while looking at a smartphone or the like. This allows the information processing device 100 to identify the second stride section 120 in the second period in which the subject has traveled in the special walking manner. Therefore, the information processing device 100 may respond to a request to identify the second stride section having the special walking manner, in addition to the normal walking, from the viewpoint of health management, accident suppression, or the like.

Here, the case where the normal walking refers to traveling by walking in a natural posture in a straight-going manner has been described, but the normal walking is not limited to this. For example, there may be a case where the normal walking is an optional walking manner. The optional walking manner may be, for example, a walking manner when the subject travels freely without being given instructions from another person, or a walking manner when the subject is injured. For example, there may be a case where the walking manner of the subject is measured at some timing, and the measured walking manner is set as a predetermined walking manner. This allows the information processing device 100 to identify the second stride section 120 in the second period in which the subject has traveled in the optional walking manner. The information processing device 100 may identify, for example, the second stride section 120 in which the subject has traveled in the same walking manner as when injured. The information processing device 100 may identify, for example, the second stride section 120 in which the subject has traveled in the same walking manner as when freely traveling without being given instructions from another person.

Here, the case where the first time-series data 101 includes the measured values regarding the movement of the right leg of the subject in the first period in each axial direction among the plurality of axial directions has been described, but the first time-series data 101 is not limited to this. For example, there may be a case where the first time-series data 101 includes the measured values regarding the movement of the left leg of the subject in the first period in each axial direction among the plurality of axial directions. For example, there may be a case where the first time-series data 101 includes the measured values regarding the movement of both legs of the subject in the first period in each axial direction among the plurality of axial directions.

Here, the case where the second time-series data 102 includes the measured values regarding the movement of the right leg of the subject in the second period in each axial direction among the plurality of axial directions has been described, but the second time-series data 102 is not limited to this. For example, there may be a case where the second time-series data 102 includes the measured values regarding the movement of the left leg of the subject in the second period in each axial direction among the plurality of axial directions. For example, there may be a case where the second time-series data 102 includes the measured values regarding the movement of both legs of the subject in the second period in each axial direction among the plurality of axial directions.

Here, the case where the information processing device 100 calculates the first index value in the first period for the first section 111 for each first stride section 110 has been described, but the information processing device 100 is not limited to this. Similarly, the case where the information processing device 100 calculates the second index value in the second period for the second section 121 for each second stride section 120 has been described, but the information processing device 100 is not limited to this.

For example, there may be a case where the information processing device 100 calculates a third index value in the first period for a third section other than the first section 111, for each first stride section 110. The third section may be a set of a plurality of sections. The third index value is, for example, an index value relating to a measured value in one axial direction based on a measured value in another axial direction, among the plurality of axial directions. Similarly, there may be a case where the information processing device 100 calculates a fourth index value in the second period for a fourth section other than the second section 121, for each second stride section 120. The fourth section may be a set of a plurality of sections. The fourth index value is, for example, an index value relating to a measured value in one axial direction based on a measured value in another axial direction, among the plurality of axial directions.

In this case, the information processing device 100 may determine whether or not each of the second stride sections 120 in the second period corresponds to the predetermined walking manner, on the basis of the third index value and the fourth index value, without using the first index value and the second index value. In addition, the information processing device 100 may determine whether or not each of the second stride sections 120 in the second period corresponds to the predetermined walking manner, on the basis of the first index value, the second index value, the third index value, and the fourth index value.

Here, the case where the first index value is the ratio of the statistical value of the measured values in the y-axis direction to the statistical value of the measured values in the x-axis direction has been described, but the first index value is not limited to this. For example, there may be a case where the first index value is the ratio of the statistical value of the measured values in the z-axis direction to the statistical value of the measured values in the x-axis direction. For example, there may be a case where the first index value is the ratio of the statistical value of the measured values in the x-axis direction to the statistical value of the measured values in the y-axis direction. For example, there may be a case where the first index value is the ratio of the statistical value of the measured values in the z-axis direction to the statistical value of the measured values in the y-axis direction.

For example, there may be a case where the information processing device 100 calculates a plurality of first index values. For example, there may be a case where the information processing device 100 calculates both of the ratio of the statistical value of the measured values in the y-axis direction to the statistical value of the measured values in the x-axis direction and the ratio of the statistical value of the measured values in the z-axis direction to the statistical value of the measured values in the x-axis direction.

Here, the case where the second index value is the ratio of the statistical value of the measured values in the y-axis direction to the statistical value of the measured values in the x-axis direction has been described, but the second index value is not limited to this. For example, there may be a case where the second index value is the ratio of the statistical value of the measured values in the z-axis direction to the statistical value of the measured values in the x-axis direction. For example, there may be a case where the second index value is the ratio of the statistical value of the measured values in the x-axis direction to the statistical value of the measured values in the y-axis direction. For example, there may be a case where the second index value is the ratio of the statistical value of the measured values in the z-axis direction to the statistical value of the measured values in the y-axis direction.

For example, there may be a case where the information processing device 100 calculates a plurality of second index values. For example, there may be a case where the information processing device 100 calculates both of the ratio of the statistical value of the measured values in the y-axis direction to the statistical value of the measured values in the x-axis direction and the ratio of the statistical value of the measured values in the z-axis direction to the statistical value of the measured values in the x-axis direction.

Here, the case where the information processing device 100 works independently has been described, but the information processing device 100 is not limited to this. For example, there may be a case where the information processing device 100 cooperates with another computer. For example, the information processing device 100 may provide, for example, the measured values in each axial direction among the plurality of axial directions in the identified second stride section 120 to another computer that analyzes the health condition of the subject.

(Example of Information Processing System 200)

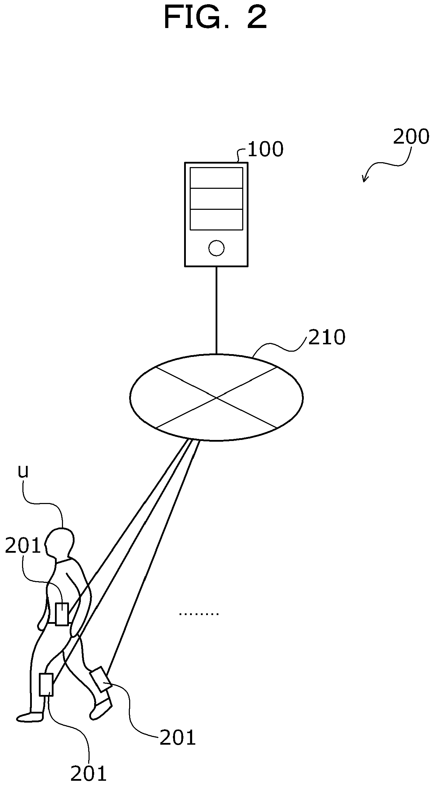

Next, an example of an information processing system 200 to which the information processing device 100 illustrated in FIG. 1 is applied will be described with reference to FIG. 2.

FIG. 2 is an explanatory diagram illustrating an example of the information processing system 200. In FIG. 2, the information processing system 200 includes the information processing device 100 and information measuring devices 201.

In the information processing system 200, the information processing device 100 and the information measuring devices 201 are connected via a wired or wireless network 210. For example, the network 210 is a local area network (LAN), a wide area network (WAN), the Internet, or the like.

The information processing device 100 receives the measured values regarding the movement of the legs of the subject in the first period, from the information measuring devices 201. The first period is a period in which the subject is deemed to have walked normally. The measured values are, for example, feature values measured in each axial direction among the x-, y-, and z-axis directions. The feature value is, for example, an angular velocity. The information processing device 100 receives, from the information measuring devices 201, for example, the angular velocity regarding the movement of the legs of the subject measured in each axial direction among the x-, y-, and z-axis directions, which is associated with information indicating that the subject walked normally.

The information processing device 100 generates the first time-series data including the received measured values. On the basis of the generated first time-series data, the information processing device 100 calculates the first index value in the first period for the first section in which the subject swings one leg, for each first stride section. For example, the first index value is the ratio of the statistical value of the measured values in the y-axis direction to the statistical value of the measured values in the x-axis direction. For example, on the basis of the first time-series data, the information processing device 100 calculates, as the first index value, the ratio of the statistical value of the measured values in the y-axis direction to the statistical value of the measured values in the x-axis direction in the first period for the first section for each first stride section.

The information processing device 100 receives the measured values regarding the movement of the legs of the subject in the second period, from the information measuring devices 201. The second period is a period different from the first period and is a target to be determined as to whether or not a section corresponding to the normal walking is included. The measured values are, for example, feature values measured in each axial direction among the x-, y-, and z-axis directions. The feature value is, for example, an angular velocity. The information processing device 100 receives, from the information measuring devices 201, for example, the angular velocity regarding the movement of the legs of the subject measured in each axial direction among the x-, y-, and z-axis directions.

The information processing device 100 generates the second time-series data including the received measured values. On the basis of the generated second time-series data, the information processing device 100 calculates the second index value in the second period for the second section in which the subject swings one leg, for each second stride section. For example, the second index value is the ratio of the statistical value of the measured values in the y-axis direction to the statistical value of the measured values in the x-axis direction. For example, on the basis of the second time-series data, the information processing device 100 calculates, as the second index value, the ratio of the statistical value of the measured values in the y-axis direction to the statistical value of the measured values in the x-axis direction in the second period for the second section for each second stride section.

The information processing device 100 determines whether or not each of the second stride sections in the second period corresponds to the normal walking, on the basis of the calculated first index value and the calculated second index value. The information processing device 100 analyzes the health condition of the subject on the basis of the measured values regarding the movement of the legs of the subject in each axial direction in the second stride section determined to correspond to the normal walking. The information processing device 100 outputs the result of the analysis such that the user is allowed to refer to the output result. For example, the information processing device 100 is a server, a personal computer (PC), or the like.

The information measuring device 201 is a computer attached to the subject. The subject is, for example, a person u. The information measuring device 201 includes, for example, a sensor device. The sensor device includes, for example, a gyro sensor that measures the angular velocity. The information measuring device 201 acquires the measured values regarding the movement of the leg of the subject in each axial direction among the x-, y-, and z-axis directions by the sensor device. The information measuring device 201 acquires, for example, the angular velocity regarding the movement of the leg of the subject measured in each axial direction among the x-, y-, and z-axis directions by the sensor device.

The information measuring device 201 is capable of determining whether or not the subject is walking normally. The information measuring device 201 sets that the subject is walking normally, for example, on the basis of an operation input from the subject. The information measuring device 201 transmits the acquired measured values to the information processing device 100. For example, if the subject is set to be walking normally, the information measuring device 201 transmits the acquired measured values to the information processing device 100 in association with the information indicating that the subject walked normally. For example, the information measuring device 201 transmits the acquired angular velocity to the information processing device 100. For example, the information measuring device 201 is a smartphone, a wearable terminal, or the like.

Here, the case where the information processing device 100 receives, from the information measuring devices 201, the measured values regarding the movement of the legs of the subject in each axial direction among the x-, y-, and z-axis directions, which are associated with the information indicating that the subject walked normally, has been described, but the information processing device 100 is not limited to this. For example, there may be a case where, when receiving the measured values regarding the movement of the legs of the subject in each axial direction among the x-, y-, and z-axis directions on the basis of an operation input from the user, the information processing device 100 determines whether or not the received measured values correspond to the normal walking.

Here, the case where the information processing device 100 and the information measuring devices 201 are different devices has been described, but the information processing device 100 is not limited to this. For example, there may be a case where the information processing device 100 has a function as the information measuring device 201 and works also as the information measuring device 201.

(Hardware Configuration Example of Information Processing Device 100)

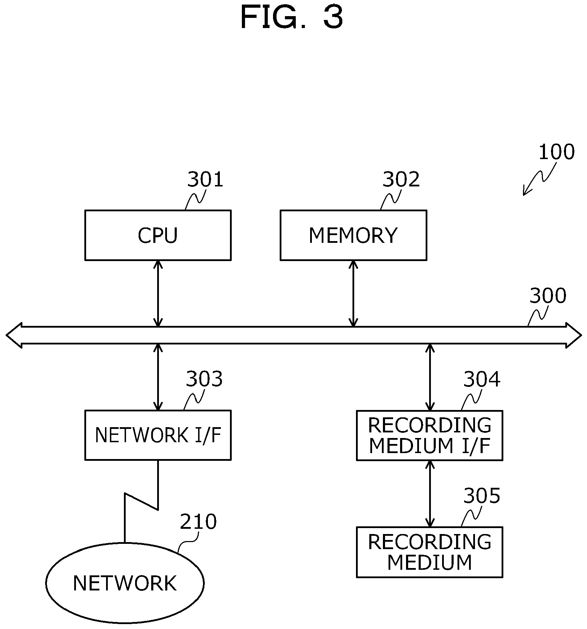

Next, a hardware configuration example of the information processing device 100 will be described with reference to FIG. 3.

FIG. 3 is a block diagram illustrating a hardware configuration example of the information processing device 100. In FIG. 3, the information processing device 100 includes a central processing unit (CPU) 301, a memory 302, a network interface (I/F) 303, a recording medium I/F 304, and a recording medium 305. In addition, the individual components are connected to each other by a bus 300.

Here, the CPU 301 is responsible for overall control of the information processing device 100. For example, the memory 302 includes a read only memory (ROM), a random access memory (RAM), a flash ROM, and the like. For example, the flash ROM or the ROM stores various programs, and the RAM is used as a work area for the CPU 301. The programs stored in the memory 302 are loaded into the CPU 301 to cause the CPU 301 to execute coded processing.

The network I/F 303 is connected to the network 210 through a communication line and is connected to another computer via the network 210. Then, the network I/F 303 is responsible for an interface between the network 210 and the inside and controls input and output of data from and to another computer. For example, the network I/F 303 is a modem, a LAN adapter, or the like.

The recording medium I/F 304 controls read and write of data from and to the recording medium 305 under the control of the CPU 301. For example, the recording medium I/F 304 is a disk drive, a solid state drive (SSD), a universal serial bus (USB) port, or the like. The recording medium 305 is a nonvolatile memory that stores data written under the control of the recording medium I/F 304. For example, the recording medium 305 is a disk, a semiconductor memory, a USB memory, or the like. The recording medium 305 may be attachable to and detachable from the information processing device 100.

For example, the information processing device 100 may include a keyboard, a mouse, a display, a printer, a scanner, a microphone, a speaker, or the like, in addition to the components described above. In addition, the information processing device 100 may include a plurality of the recording medium I/Fs 304 and the recording media 305. Furthermore, the information processing device 100 does not have to include the recording medium I/F 304 or the recording medium 305.

(Hardware Configuration Example of Information Measuring Device 201)

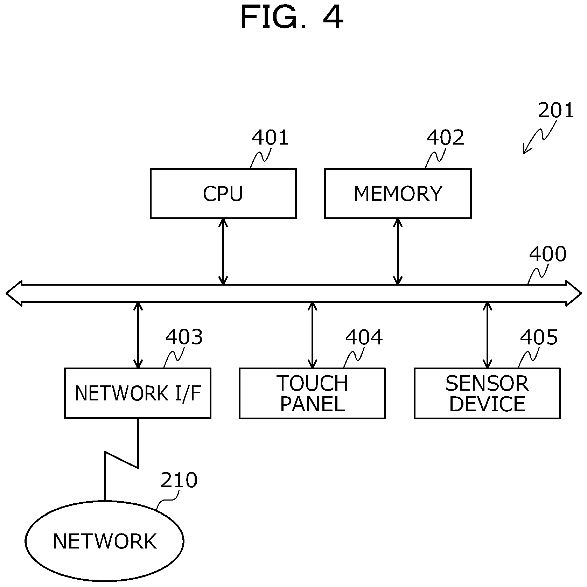

Next, a hardware configuration example of the information measuring device 201 included in the information processing system 200 illustrated in FIG. 2 will be described with reference to FIG. 4.

FIG. 4 is a block diagram illustrating a hardware configuration example of the information measuring device 201. In FIG. 4, the information measuring device 201 includes a CPU 401, a memory 402, a network I/F 403, a touch panel 404, and a sensor device 405. In addition, the individual components are connected to each other by a bus 400.

Here, the CPU 401 is responsible for overall control of the information measuring device 201. For example, the memory 402 includes a ROM, a RAM, a flash ROM, and the like. For example, the flash ROM or the ROM stores various programs, and the RAM is used as a work area for the CPU 401. The programs stored in the memory 402 are loaded into the CPU 401 to cause the CPU 401 to execute coded processing.

The network I/F 403 is connected to the network 210 through a communication line and is connected to another computer via the network 210. Then, the network I/F 403 is responsible for an interface between the network 210 and the inside and controls input and output of data from and to another computer.

For example, the network I/F 403 is a communication circuit having an antenna for third generation (3G), a communication circuit having an antenna for long term evolution (LTE), or the like. The network I/F 403 may be a communication circuit having an antenna for Wi-Fi (registered trademark).

The touch panel 404 has a display that displays data such as a document, an image, or function information, as well as a cursor, an icon, or a toolbox. The touch panel 404 is provided on the display or on an outer peripheral portion of the display and has a detection device that detects a contact position of a user on the touch panel 404. For example, the detection device detects the contact position, using a resistance film scheme, a capacitance scheme, an ultrasonic scheme, an optical scheme, an electromagnetic induction scheme, or the like. The touch panel 404 inputs characters, numbers, various instructions, and the like according to the contact position of the user.

The sensor device 405 detects the state of the information measuring device 201 corresponding to the state of the subject. The sensor device 405 detects, for example, at least one of the position, movement, and orientation of the information measuring device 201. For example, the sensor device 405 includes a gyro sensor. In addition, the sensor device 405 may include an acceleration sensor, a vibration sensor, or the like. Furthermore, the sensor device 405 may include a GPS receiver and detect the GPS coordinates of the information measuring device 201.

For example, the information measuring device 201 may include a disk drive, a disk, an SSD, a semiconductor memory, a scanner, a printer, or the like, in addition to the components described above.

(Functional Configuration Example of Information Processing Device 100)

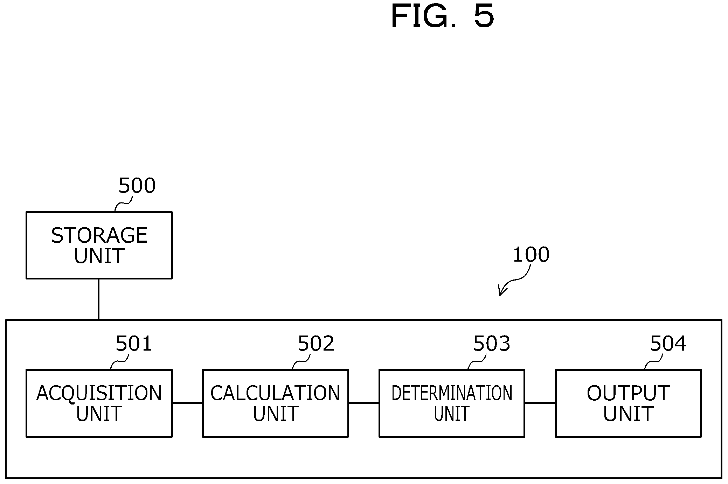

Next, a functional configuration example of the information processing device 100 will be described with reference to FIG. 5.

FIG. 5 is a block diagram illustrating a functional configuration example of the information processing device 100. The information processing device 100 includes a storage unit 500, an acquisition unit 501, a calculation unit 502, a determination unit 503, and an output unit 504.

For example, the storage unit 500 is implemented by a storage area such as the memory 302 or the recording medium 305 illustrated in FIG. 3. Hereinafter, the case where the storage unit 500 is included in the information processing device 100 will be described, but the storage unit 500 is not limited to this. For example, there may be a case where the storage unit 500 is included in a device different from the information processing device 100, and the information processing device 100 is allowed to refer to stored contents of the storage unit 500.

The acquisition unit 501 to the output unit 504 function as an example of a control unit. For example, the acquisition unit 501 to the output unit 504 implement functions thereof by causing the CPU 301 to execute a program stored in a storage area such as the memory 302 or the recording medium 305 illustrated in FIG. 3 or by the network I/F 303. A processing result of each functional unit is stored in, for example, a storage area such as the memory 302 or the recording medium 305 illustrated in FIG. 3.

The storage unit 500 stores various types of information referred to or updated in the processing of each functional unit. The storage unit 500 stores the first time-series data. The first time-series data includes, for example, measured values regarding the movement of the legs of the subject in the first period in each axial direction among a plurality of axial directions. The first period is a period in which the subject has traveled in a predetermined walking manner. The predetermined walking manner corresponds to, for example, a walking manner in the normal walking. The plurality of axial directions includes the front-back direction, the left-right direction, and the up-down direction of the subject. The measured value is a value regarding the movement of the leg. The measured value is, for example, the angular velocity of the leg. The measured value may be the angular velocity of the waist or the arm as long as the value is regarding the movement of the leg. For example, the first time-series data includes angular velocity regarding the movement of the legs of the subject in the first period in each axial direction. The first time-series data is acquired by the acquisition unit 501 and stored by the storage unit 500, for example.

The storage unit 500 stores the second time-series data. The second time-series data includes, for example, measured values regarding the movement of the legs of the subject in the second period in each axial direction among the plurality of axial directions. The second period is a period different from the first period. The plurality of axial directions includes the front-back direction, the left-right direction, and the up-down direction of the subject. The measured value is a value regarding the movement of the leg. The measured value is, for example, the angular velocity of the leg. The measured value may be the angular velocity of the waist or the arm as long as the value is regarding the movement of the leg. For example, the second time-series data includes angular velocity regarding the movement of the legs of the subject in the second period in each axial direction. The second time-series data is acquired by the acquisition unit 501 and stored by the storage unit 500, for example.

The storage unit 500 may store the first index value in the first period for the first section in which the subject swings one leg, for each first stride section corresponding to one step of the subject. The first index value is, for example, an index value relating to a measured value in one axial direction based on a measured value in another axial direction, among the plurality of axial directions. For example, the first index value is the ratio of the statistical value of the measured values in one axial direction to the statistical value of the measured values in another axial direction, among the plurality of axial directions. The first index value is calculated by the calculation unit 502 and stored by the storage unit 500, for example.

The storage unit 500 may store the second index value in the second period for the second section in which the subject swings one leg, for each second stride section corresponding to one step of the subject. The second index value is, for example, an index value relating to a measured value in one axial direction based on a measured value in another axial direction, among the plurality of axial directions. For example, the second index value is the ratio of the statistical value of the measured values in one axial direction to the statistical value of the measured values in another axial direction, among the plurality of axial directions. The second index value is calculated by the calculation unit 502 and stored by the storage unit 500, for example.

The storage unit 500 may store the third index value in the first period for the third section other than the first section, for each first stride section. The third index value is, for example, an index value relating to a measured value in one axial direction based on a measured value in another axial direction, among the plurality of axial directions. For example, the third index value is the ratio of the statistical value of the measured values in one axial direction to the statistical value of the measured values in another axial direction, among the plurality of axial directions. The third index value is calculated by the calculation unit 502 and stored by the storage unit 500, for example.

The storage unit 500 may store the fourth index value in the second period for the fourth section other than the second section, for each second stride section. The fourth index value is, for example, an index value relating to a measured value in one axial direction based on a measured value in another axial direction, among the plurality of axial directions. For example, the fourth index value is the ratio of the statistical value of the measured values in one axial direction to the statistical value of the measured values in another axial direction, among the plurality of axial directions. The fourth index value is calculated by the calculation unit 502 and stored by the storage unit 500, for example.

The acquisition unit 501 acquires various types of information to be used for the processing of each functional unit. The acquisition unit 501 stores the acquired various types of information in the storage unit 500 or outputs the acquired various types of information to each functional unit. In addition, the acquisition unit 501 may output the various types of information stored in the storage unit 500 to each functional unit. The acquisition unit 501 acquires the various types of information on the basis of, for example, an operation input from the user. The acquisition unit 501 may receive the various types of information from, for example, a device different from the information processing device 100.

The acquisition unit 501 acquires the first time-series data. The acquisition unit 501 acquires the first time-series data, for example, by receiving the first time-series data from another computer. The acquisition unit 501 may acquire the first time-series data, for example, by receiving the measured values regarding the movement of the legs of the subject in the first period in each axial direction among the plurality of axial directions from another computer and generating the first time-series data including the received measured values. The acquisition unit 501 may acquire the first time-series data, for example, by accepting the input of the first time-series data.

The acquisition unit 501 acquires the second time-series data. The acquisition unit 501 acquires the second time-series data, for example, by receiving the second time-series data from another computer. The acquisition unit 501 may acquire the second time-series data, for example, by receiving the measured values regarding the movement of the legs of the subject in the second period in each axial direction among the plurality of axial directions from another computer and generating the second time-series data including the received measured values. The acquisition unit 501 may acquire the second time-series data, for example, by accepting the input of the second time-series data.

The acquisition unit 501 may accept a start trigger to start the processing of any one of the functional units. The start trigger is, for example, predetermined operation input made by the user. The start trigger may be, for example, reception of predetermined information from another computer. The start trigger may be, for example, output of predetermined information by any one of the functional units.

For example, the acquisition unit 501 may accept the fact that the first time-series data has been acquired, as a start trigger to start the processing of the calculation unit 502. For example, the acquisition unit 501 may accept the fact that the second time-series data has been acquired, as a start trigger to start the processing of the calculation unit 502 and the determination unit 503.

The calculation unit 502 calculates the first index value in the first period for the first section for each first stride section, on the basis of the acquired first time-series data. For example, the calculation unit 502 calculates, as the first index value, the ratio of the statistical value of the measured values in one axial direction to the statistical value of the measured values in another axial direction among the plurality of axial directions, in the first period for the first section for each first stride section. This allows the calculation unit 502 to identify a feature that appears in the measured values in the first stride section when the subject walks normally. In addition, the calculation unit 502 may obtain information that serves as a guideline for determining whether or not the second stride section in the second period corresponds to the predetermined walking manner.

The calculation unit 502 calculates the second index value in the second period for the second section for each second stride section, on the basis of the acquired second time-series data. For example, the calculation unit 502 calculates, as the second index value, the ratio of the statistical value of the measured values in one axial direction to the statistical value of the measured values in another axial direction among the plurality of axial directions, in the second period for the second section for each second stride section. This allows the calculation unit 502 to identify a feature that appears in the measured values in the second period in the second stride section. In addition, the calculation unit 502 may obtain information that serves as a guideline for determining whether or not the second stride section in the second period corresponds to the predetermined walking manner.

The calculation unit 502 calculates the third index value in the first period for the third section other than the first section, for each first stride section, on the basis of the acquired first time-series data. For example, the calculation unit 502 calculates, as the third index value, the ratio of the statistical value of the measured values in one axial direction to the statistical value of the measured values in another axial direction among the plurality of axial directions, in the first period for the third section for each first stride section. This allows the calculation unit 502 to identify a feature that appears in the measured values in the first stride section when the subject walks normally. In addition, the calculation unit 502 may obtain information that serves as a guideline for determining whether or not the second stride section in the second period corresponds to the predetermined walking manner.

The calculation unit 502 calculates the fourth index value in the second period for the fourth section other than the second section, for each second stride section, on the basis of the acquired second time-series data. For example, the calculation unit 502 calculates, as the fourth index value, the ratio of the statistical value of the measured values in one axial direction to the statistical value of the measured values in another axial direction among the plurality of axial directions, in the second period for the fourth section for each second stride section. This allows the calculation unit 502 to identify a feature that appears in the measured values in the second period in the second stride section. In addition, the calculation unit 502 may obtain information that serves as a guideline for determining whether or not the second stride section in the second period corresponds to the predetermined walking manner.

The determination unit 503 determines whether or not each of the second stride sections in the second period corresponds to the predetermined walking manner, on the basis of the calculated first index value and the calculated second index value. For example, when the difference between the calculated first index value and the second index value calculated for any one of the second stride sections is relatively small, the determination unit 503 determines that the any one of the second stride sections corresponds to the predetermined walking manner.

For example, when the percentage of the second index value to the first index value is equal to or lower than the threshold value for any one of the second stride sections, the determination unit 503 determines that the any one of the second stride sections corresponds to the predetermined walking manner. On the other hand, for example, when the percentage of the second index value to the first index value is higher than the threshold value for any one of the second stride sections, the determination unit 503 determines that the any one of the second stride sections does not correspond to the predetermined walking manner.

This allows the determination unit 503 to accurately determine whether or not the second stride section corresponds to the predetermined walking manner. Therefore, the determination unit 503 may facilitate the use of information regarding the second stride section corresponding to the predetermined walking manner, which is useful for analyzing the health condition of the subject.

The determination unit 503 may determine whether or not each of the second stride sections in the second period corresponds to the predetermined walking manner, on the basis of the calculated third index value and the calculated fourth index value. For example, when the difference between the calculated third index value and the fourth index value calculated for any one of the second stride sections is relatively small, the determination unit 503 determines that the any one of the second stride sections corresponds to the predetermined walking manner.

For example, when the percentage of the fourth index value to the third index value is equal to or lower than the threshold value for any one of the second stride sections, the determination unit 503 determines that the any one of the second stride sections corresponds to the predetermined walking manner. On the other hand, for example, when the percentage of the fourth index value to the third index value is higher than the threshold value for any one of the second stride sections, the determination unit 503 determines that the any one of the second stride sections does not correspond to the predetermined walking manner.

This allows the determination unit 503 to accurately determine whether or not the second stride section corresponds to the predetermined walking manner. Therefore, the determination unit 503 may facilitate the use of information regarding the second stride section corresponding to the predetermined walking manner, which is useful for analyzing the health condition of the subject.

The determination unit 503 may determine whether or not each of the second stride sections in the second period corresponds to the walking manner, on the basis of the calculated first index value, the calculated second index value, the calculated third index value, and the calculated fourth index value. For example, when the difference between the first index value and the second index value is relatively small and the difference between the third index value and the fourth index value is relatively small for any one of the second stride sections, the determination unit 503 determines that the any one of the second stride sections corresponds to the predetermined walking manner.

For example, the determination unit 503 calculates a statistical value of the percentage of the second index value to the first index value and the percentage of the fourth index value to the third index value in the second period for each of the second stride sections. For example, when the calculated statistical value is equal to or lower than a predetermined threshold value for any one of the second stride sections, the determination unit 503 determines that the any one of the second stride sections corresponds to the predetermined walking manner. For example, when the calculated statistical value is higher than the predetermined threshold value for any one of the second stride sections, the determination unit 503 determines that the any one of the second stride sections does not correspond to the predetermined walking manner.

For example, the determination unit 503 determines whether or not the percentage of the second index value to the first index value is equal to or lower than a first threshold value in the second period for each of the second stride sections. In addition, for example, the determination unit 503 determines whether or not the percentage of the fourth index value to the third index value is equal to or lower than a second threshold value in the second period for each of the second stride sections.

Then, for example, the determination unit 503 determines any one of the second stride sections determined to have the percentage of the second index value to the first index value equal to or lower than the first threshold value and the percentage of the fourth index value to the third index value equal to or lower than the second threshold value to correspond to the predetermined walking manner. On the other hand, for example, the determination unit 503 determines any one of the second stride sections determined to have the percentage of the second index value to the first index value higher than the first threshold value to not correspond to the predetermined walking manner. In addition, for example, the determination unit 503 determines any one of the second stride sections determined to have the percentage of the fourth index value to the third index value higher than the second threshold value to not correspond to the predetermined walking manner.

This allows the determination unit 503 to accurately determine whether or not the second stride section corresponds to the predetermined walking manner. Therefore, the determination unit 503 may facilitate the use of information regarding the second stride section corresponding to the predetermined walking manner, which is useful for analyzing the health condition of the subject.

The output unit 504 outputs a processing result of at least any one of the functional units. An output format is, for example, display on a display, print output to a printer, transmission to an external device by the network I/F 303, or storage in a storage area such as the memory 302 or the recording medium 305. Consequently, the output unit 504 may allow the user to be notified of the processing result of at least any one of the functional units and may improve convenience of the information processing device 100.

The output unit 504 outputs, for example, information indicating the second stride section determined to correspond to the predetermined walking manner. For example, the output unit 504 outputs the information indicating the second stride section determined to correspond to the predetermined walking manner such that the user is allowed to refer to the output information. Consequently, the output unit 504 may allow the user to easily grasp the second stride section corresponding to the predetermined walking manner, which is useful for analyzing the health condition of the subject.

The output unit 504 may output, for example, not only the information indicating the second stride section determined to correspond to the predetermined walking manner, but also the measured values regarding the movement of the legs of the subject in that second stride section in each axial direction among the plurality of axial directions. For example, the output unit 504 outputs not only the information indicating the second stride section determined to correspond to the predetermined walking manner, but also the measured values in that second stride section in each axial direction such that the user is allowed to refer to the output information and measured values. Consequently, the output unit 504 may allow the user to easily grasp the second stride section corresponding to the predetermined walking manner, and the measured values in that second stride section in each axial direction, which are useful for analyzing the health condition of the subject.

For example, the output unit 504 may output the measured values in each axial direction in the second stride section determined to correspond to the predetermined walking manner to an analysis unit that analyzes the health condition of the subject. For example, the analysis unit may be included in the information processing device 100. The analysis unit may be included in another computer. Consequently, the output unit 504 may enable accurate analysis of the health condition of the subject.

(Specific Functional Configuration Example of Information Processing Device 100)

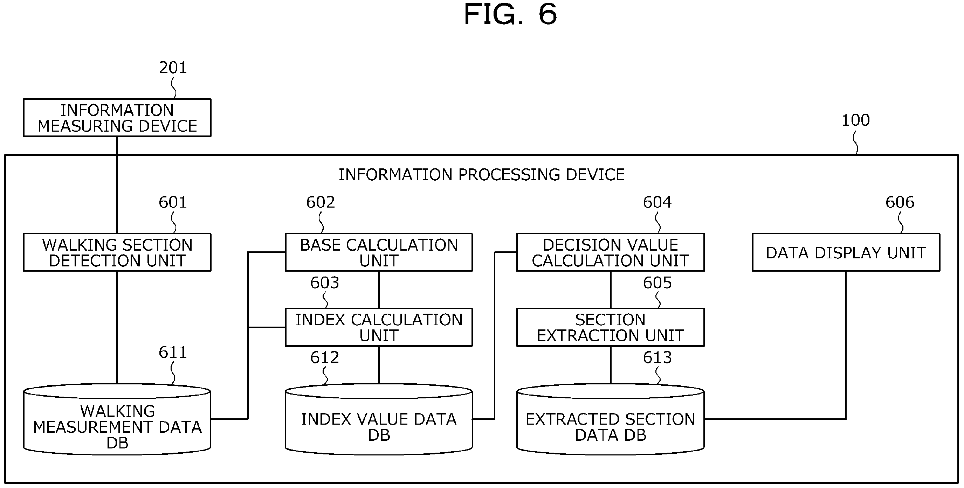

Next, a specific functional configuration example of the information processing device 100 will be described with reference to FIG. 6.

FIG. 6 is a block diagram illustrating a specific functional configuration example of the information processing device 100. The information processing device 100 includes a walking section detection unit 601, a base calculation unit 602, an index calculation unit 603, a decision value calculation unit 604, a section extraction unit 605, and a data display unit 606. In addition, the information processing device 100 includes a walking measurement data database (DB) 611, an index value data DB 612, and an extracted section data DB 613.

The walking section detection unit 601 receives, from the information measuring devices 201, the measured values regarding the legs of the subject in each direction among the x-, y-, and z-axis directions. The walking section detection unit 601 generates walking measurement data summarizing the received measured values during a time span deemed to be a walking section in which the subject walked.

Walking is, for example, a state in which the subject is traveling using own legs of the subject. Walking includes going straight, changing the direction, or the like. The walking section is a time span from when the subject swings one leg and begins walking to when the subject finishes walking. The walking measurement data is time-series data of the measured values.

For example, the walking section detection unit 601 generates the walking measurement data when the subject walks normally for a walking test. The walking test is to go straight for 10 m in a natural posture. In the following description, the walking measurement data when the subject walks normally will be sometimes referred to as "walking measurement data of the walking test".

For example, the walking section detection unit 601 generates the walking measurement data when the subject walks in a daily way. The daily walking is walking other than the walking test. The daily walking is, for example, traveling by the subject at home or outdoors. In the following description, the walking measurement data during the daily walking will be sometimes referred to as "walking measurement data of the daily walking". The walking section detection unit 601 stores the generated walking measurement data in the walking measurement data DB 611. The walking measurement data DB 611 stores the walking measurement data.

The base calculation unit 602 reads the walking measurement data of the walking test from the walking measurement data DB 611. The base calculation unit 602 calculates an average value of the measured values in each axial direction for each stride section at the time of normal walking, on the basis of the walking measurement data of the walking test.

The base calculation unit 602 calculates the ratio of the average value of the measured values in the y-axis direction to the average value of the measured values in the x-axis direction, as an index value relating to the y-axis direction, for each stride section at the time of normal walking. The base calculation unit 602 calculates the ratio of the average value of the measured values in the z-axis direction to the average value of the measured values in the x-axis direction, as an index value relating to the z-axis direction, for each stride section at the time of normal walking.

The base calculation unit 602 calculates the average value of the index values relating to the y-axis direction in the whole plurality of stride sections at the time of normal walking, as a base index relating to the y-axis direction. The base calculation unit 602 calculates the average value of the index values relating to the z-axis direction in the whole plurality of stride sections at the time of normal walking, as a base index relating to the z-axis direction.

The base calculation unit 602 stores the calculated base index relating to the y-axis direction and the calculated base index relating to the z-axis direction in the index value data DB 612. The index value data DB 612 stores the base index relating to the y-axis direction and the base index relating to the z-axis direction.

The index calculation unit 603 reads the walking measurement data of the daily walking from the walking measurement data DB 611. The index calculation unit 603 calculates an average value of the measured values in each axial direction for each stride section at the time of daily walking, on the basis of the walking measurement data of the daily walking.

The index calculation unit 603 calculates the ratio of the average value of the measured values in the y-axis direction to the average value of the measured values in the x-axis direction, as an index value relating to the y-axis direction, for each stride section at the time of daily walking. The index calculation unit 603 calculates the ratio of the average value of the measured values in the z-axis direction to the average value of the measured values in the x-axis direction, as an index value relating to the z-axis direction, for each stride section at the time of daily walking.

The index calculation unit 603 stores the calculated index values relating to the y-axis direction and the calculated index values relating to the z-axis direction in the index value data DB 612 in association with the stride sections at the time of daily walking for each stride section. The index value data DB 612 stores the index values relating to the y-axis direction and the index values relating to the z-axis direction in association with the stride sections, for each stride section at the time of daily walking.

The decision value calculation unit 604 reads the base index relating to the y-axis direction and the base index relating to the z-axis direction from the index value data DB 612. The decision value calculation unit 604 reads the index values relating to the y-axis direction and the index values relating to the z-axis direction associated with the stride sections, for each stride section at the time of walking test, from the index value data DB 612.

The decision value calculation unit 604 calculates the percentage of the read index value relating to the y-axis direction to the read base index relating to the y-axis direction, for each stride section at the time of walking test. The decision value calculation unit 604 calculates the percentage of the read index value relating to the z-axis direction to the read base index relating to the z-axis direction, for each stride section at the time of walking test. The decision value calculation unit 604 calculates an average value of the calculated percentages, as a decision value for each stride section at the time of walking test.

The section extraction unit 605 determines whether or not the calculated decision value is equal to or lower than a predetermined threshold value, for each stride section at the time of walking test. The section extraction unit 605 extracts a stride section determined to have a decision value equal to or lower than the predetermined threshold value, as a normal walking section, from among the stride sections at the time of walking test. The normal walking section is a section in which the subject is deemed to have walked normally. The section extraction unit 605 stores the extracted normal walking section in the extracted section data DB 613. The extracted section data DB 613 stores the normal walking section.

The data display unit 606 reads the normal walking section from the extracted section data DB 613. The data display unit 606 displays the normal walking section on a display. The data display unit 606 may display the normal walking section and the measured values in each axial direction in the normal walking section on the display in association with each other.

This allows the information processing device 100 to identify the stride section in which the subject walks normally. Therefore, the information processing device 100 may identify, for example, the measured values in each axial direction in the stride section in which the subject walked normally, which are useful information when the health condition of the subject is analyzed. The information processing device 100 may facilitate the analysis of the health condition of the subject, for example.

(Action Example of Information Processing Device 100)

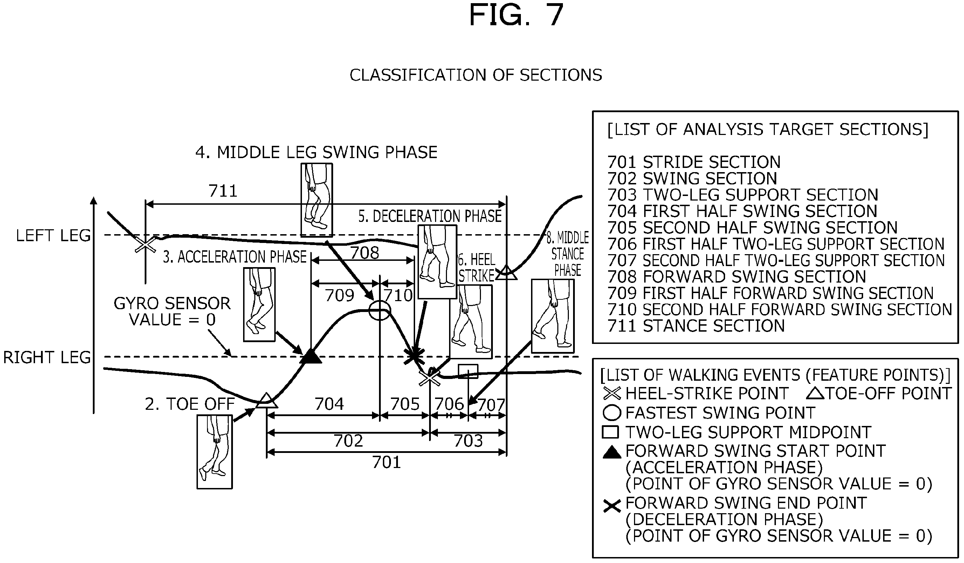

Next, an action example of the information processing device 100 will be described with reference to FIGs. 7 to 16. First, an example of classification of a plurality of sections forming a stride section will be described with reference to FIG. 7, for example.

FIG. 7 is an explanatory diagram illustrating an example of classification of a plurality of sections. In FIG. 7, for the right leg, a stride section 701 is formed by a swing section 702 and a two-leg support section 703. The swing section 702 is formed by a first half swing section 704 and a second half swing section 705. The two-leg support section 703 is formed by a first half two-leg support section 706 and a second half two-leg support section 707. The swing section 702 includes a forward swing section 708. The forward swing section 708 is formed by a first half forward swing section 709 and a second half forward swing section 710. The stride section 701 is included in a stance section 711.

The stance section 711 is a section from a heel-strike point of the left leg to a toe-off point of the left leg. The stride section 701 is a section from the toe-off point of the right leg to the toe-off point of the left leg. The swing section 702 is a section from the toe-off point of the right leg to the heel-strike point of the right leg. The two-leg support section 703 is a section from the heel-strike point of the right leg to the toe-off point of the left leg.

The first half swing section 704 is a section from the toe-off point of the right leg to the fastest swing point where the gyro sensor value in a middle leg swing phase locally becomes maximum. The second half swing section 705 is a section from the fastest swing point to the heel-strike point of the right leg. The first half two-leg support section 706 is a section from the heel-strike point of the right leg to the midpoint of the two-leg support section 703, which is a middle stance phase. The second half two-leg support section 707 is a section from the midpoint of the two-leg support section 703, which is the middle stance phase, to the toe-off point of the left leg.

The forward swing section 708 is a section from a forward swing start point where the gyro sensor value becomes zero in the first half swing section 704 to a forward swing end point where the gyro sensor value becomes zero in the second half swing section 705. The forward swing start point is an acceleration phase. The forward swing end point is a deceleration phase. The first half forward swing section 709 is a section from the forward swing start point where the gyro sensor value becomes zero in the first half swing section 704 to the fastest swing point. The second half forward swing section 710 is a section from the fastest swing point to the forward swing end point where the gyro sensor value becomes zero in the second half swing section 705.

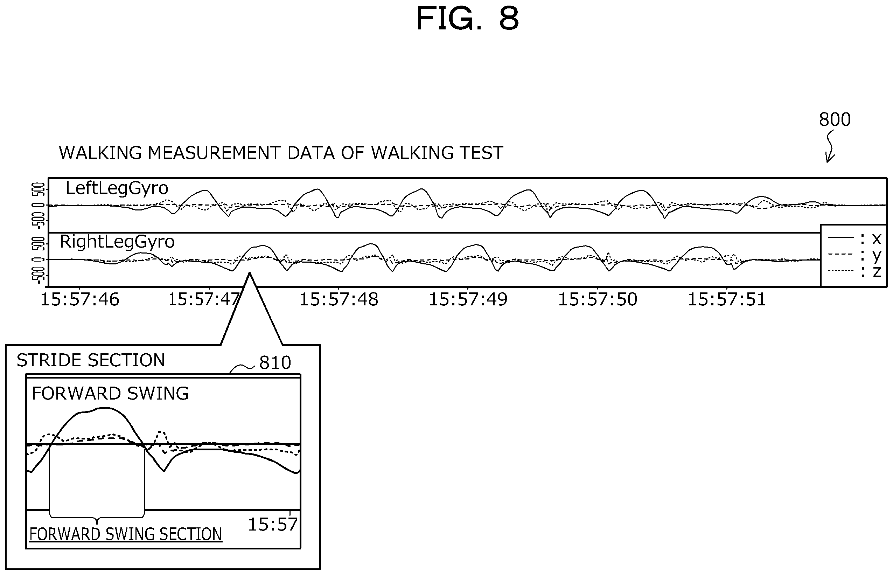

Next, an example in which the information processing device 100 calculates the base index value will be described with reference to FIGs. 8 to 11.

FIGs. 8 to 11 are explanatory diagrams illustrating an example of calculating the base index value. In FIG. 8, the information processing device 100 receives, from the information measuring devices 201, the measured values regarding the legs of the subject who walked normally for the walking test, in each direction among the x-, y-, and z-axis directions. The information processing device 100 identifies a time span deemed to be the normal walking section in which the subject walks normally, on the basis of the received measured values.

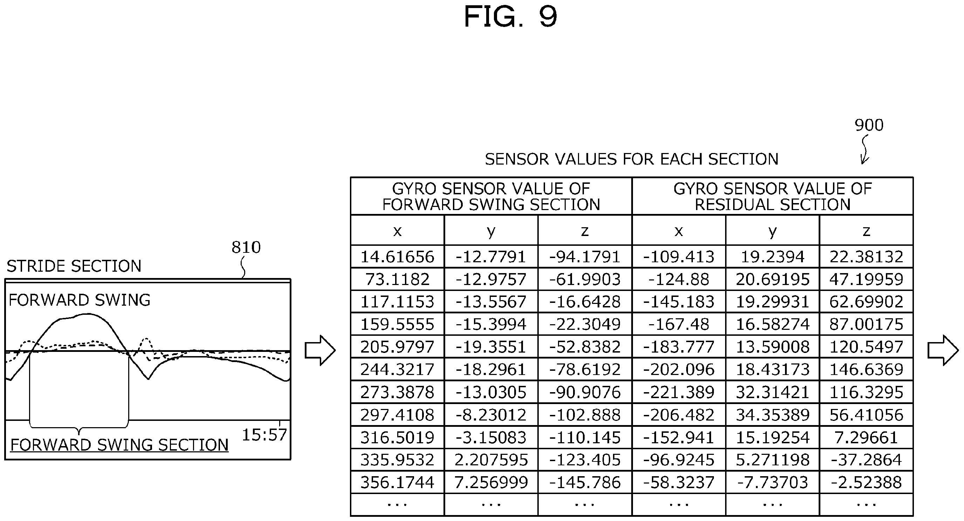

The information processing device 100 generates walking measurement data 800 of the walking test, which summarizes the received measured values, for the identified time span. The walking measurement data 800 of the walking test includes the measured values in each stride section 810. The stride section 810 includes the forward swing section. Next, description of FIG. 9 will be made.

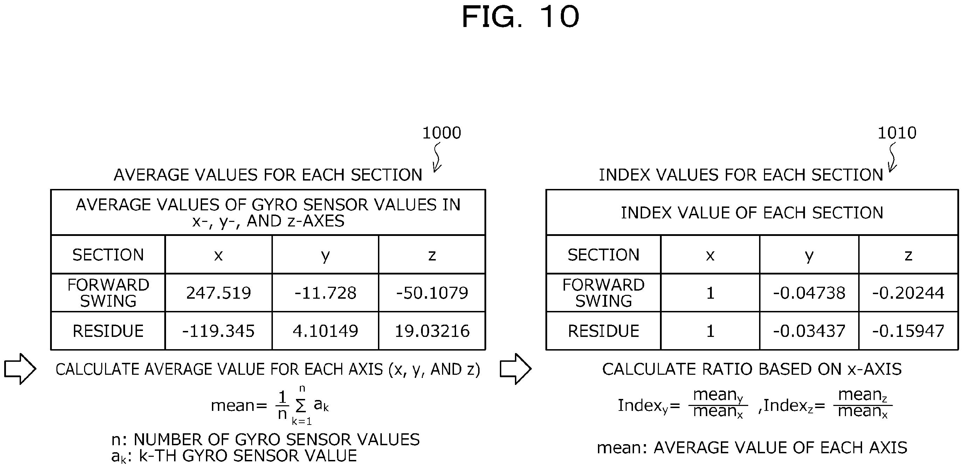

In FIG. 9, the information processing device 100 identifies a plurality of stride sections 810 on the basis of the walking measurement data 800 of the walking test. As illustrated in table 900, the information processing device 100 extracts the gyro sensor value of each axial direction among the x-, y-, and z-axis directions in the forward swing section for each stride section 810, from the walking measurement data 800 of the walking test. As illustrated in table 900, the information processing device 100 extracts the gyro sensor value of each axial direction among the x-, y-, and z-axis directions in other than the forward swing section for each stride section 810, from the walking measurement data 800 of the walking test. Next, description of FIG. 10 will be made.

In FIG. 10, as illustrated in table 1000, the information processing device 100 calculates the average value of the gyro sensor values of each axial direction among the x-, y-, and z-axis directions in the forward swing section for each stride section 810, on the basis of the extracted gyro sensor values, on the basis of following formula (1). As illustrated in table 1000, the information processing device 100 calculates the average value of the gyro sensor values of each axial direction among the x-, y-, and z-axis directions in other than the forward swing section for each stride section 810, on the basis of the extracted gyro sensor values, on the basis of following formula (1).

$$\text{mean} = 1/n\Sigma_{k=1}^{n}a_k \ \ldots \ (1)$$

The average value is denoted by mean. The number of gyro sensor values of each axial direction among the x-, y-, and z-axis directions in the forward swing section or other than the forward swing section is denoted by n. The k-th gyro sensor value of each axial direction among the x-, y-, and z-axis directions in the forward swing section or other than the forward swing section is denoted by $a_k$. It is premised that k = 1 to n holds.

As illustrated in table 1010, the information processing device 100 calculates the ratio of the average value of the gyro sensor values of each axial direction among the y- and z-axis directions to the average value of the gyro sensor values of the x-axis direction in the forward swing section for each stride section 810. The information processing device 100 calculates the ratio of the average value of the gyro sensor values of each axial direction among the y- and z-axis directions to the average value of the gyro sensor values of the x-axis direction in the forward swing section for each stride section 810, for example, on the basis of following formulas (2) and (3).

As illustrated in table 1010, the information processing device 100 calculates the ratio of the average value of the gyro sensor values of each axial direction among the y- and z-axis directions to the average value of the gyro sensor values of the x-axis direction in other than the forward swing section for each stride section 810. The information processing device 100 calculates the ratio of the average value of the gyro sensor values of each axial direction among the y- and z-axis directions to the average value of the gyro sensor values of the x-axis direction in other than the forward swing section for each stride section 810, for example, on the basis of following formulas (2) and (3).

$$\text{Index}_y = \text{mean}_y/\text{mean}_x \ \ldots \ (2)$$

$$\text{Index}_z = \text{mean}_z/\text{mean}_x \ \ldots \ (3)$$

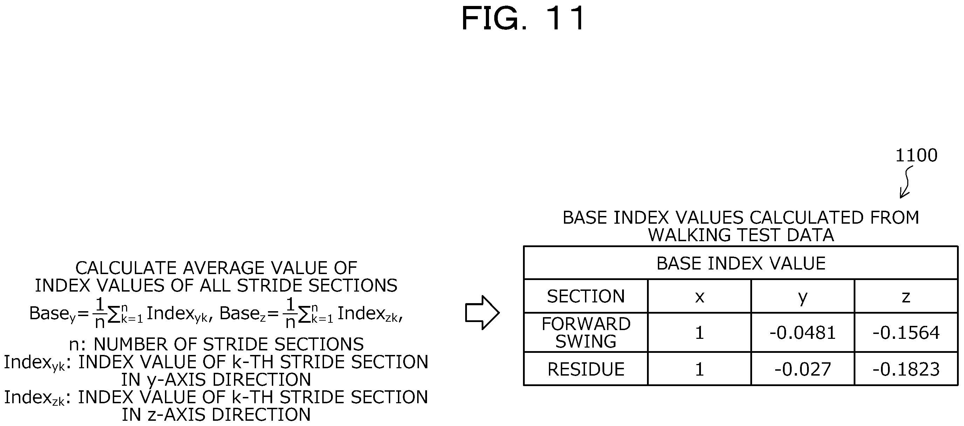

The ratio of the average value of the gyro sensor values of the y-axis direction to the average value of the gyro sensor values of the x-axis direction is denoted by $\text{Index}_y$. The ratio of the average value of the gyro sensor values of the z-axis direction to the average value of the gyro sensor values of the x-axis direction is denoted by $\text{Index}_z$. The average value of the gyro sensor values of the x-axis direction is denoted by $\text{mean}_x$. The average value of the gyro sensor values of the y-axis direction is denoted by $\text{mean}_y$. The average value of the gyro sensor values of the z-axis direction is denoted by $\text{mean}_z$. Next, description of FIG. 11 will be made.

In FIG. 11, on the basis of following formula (4), the information processing device 100 calculates the average value of the ratios of the average values of the gyro sensor values of the y-axis direction to the average values of the gyro sensor values of the x-axis direction in the whole plurality of stride sections 810. As illustrated in table 1100, the information processing device 100 sets the calculated average value as the base index value relating to the y-axis direction.

On the basis of following formula (5), the information processing device 100 calculates the average value of the ratios of the average values of the gyro sensor values of the z-axis direction to the average values of the gyro sensor values of the x-axis direction in the whole plurality of stride sections 810. As illustrated in table 1100, the information processing device 100 sets the calculated average value as the base index value relating to the z-axis direction.

$$\text{Base}_y = 1/n\sum_{k=1}^{n}\text{Index}_{yk} \ ... \ (4)$$

$$\text{Base}_z = 1/n\sum_{k=1}^{n}\text{Index}_{zk} \ ... \ (5)$$

The average value of the ratios of the average values of the gyro sensor values of the y-axis direction to the average values of the gyro sensor values of the x-axis direction is denoted by $\text{Base}_y$. The average value of the ratios of the average values of the gyro sensor values of the z-axis direction to the average values of the gyro sensor values of the x-axis direction is denoted by $\text{Base}_z$. The number of stride sections 810 is denoted by n. The ratio of the average value of the gyro sensor values of the y-axis direction to the average value of the gyro sensor values of the x-axis direction for the k-th stride section 810 is denoted by $\text{Index}_{yk}$. The ratio of the average value of the gyro sensor values of the z-axis direction to the average value of the gyro sensor values of the x-axis direction for the k-th stride section 810 is denoted by $\text{Index}_{zk}$. It is premised that k = 1 to n holds.

This allows the information processing device 100 to calculate the base index value indicating what kind of feature appears in the measured values in the stride section when the subject walks normally. Therefore, the information processing device 100 may obtain, from the walking measurement data when the subject walks in a daily way, information that serves as a guideline for identifying a stride section that is a section in which the subject walks normally, in a period in which the subject walks in a daily way.

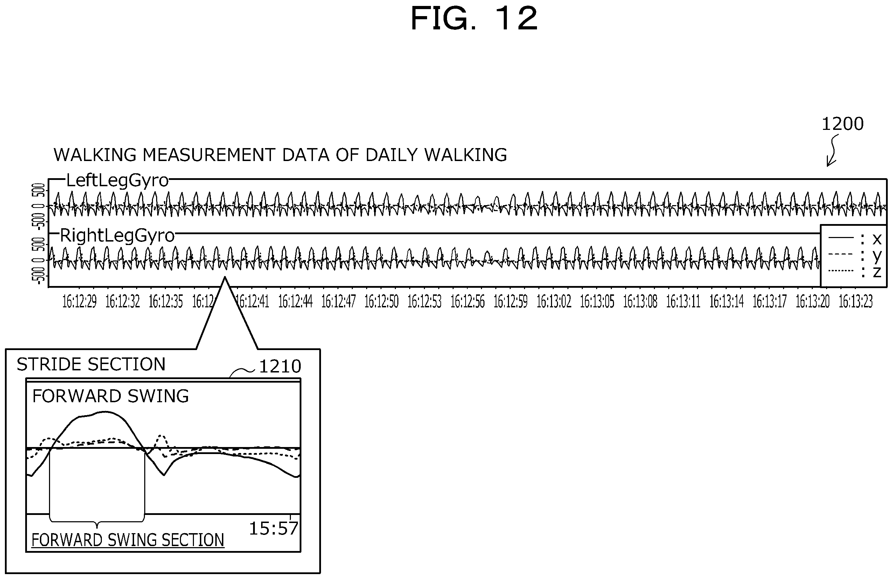

Next, an example in which the information processing device 100 calculates the index value of each stride section will be described with reference to FIGs. 12 to 14.

FIGs. 12 to 14 are explanatory diagrams illustrating an example of calculating the index value of each stride section. In FIG. 12, the information processing device 100 receives, from the information measuring devices 201, the measured values regarding the legs of the subject who walked in a daily way, in each direction among the x-, y-, and z-axis directions. The information processing device 100 identifies a time span deemed to be the daily walking section in which the subject walks in a daily way, on the basis of the received measured values.

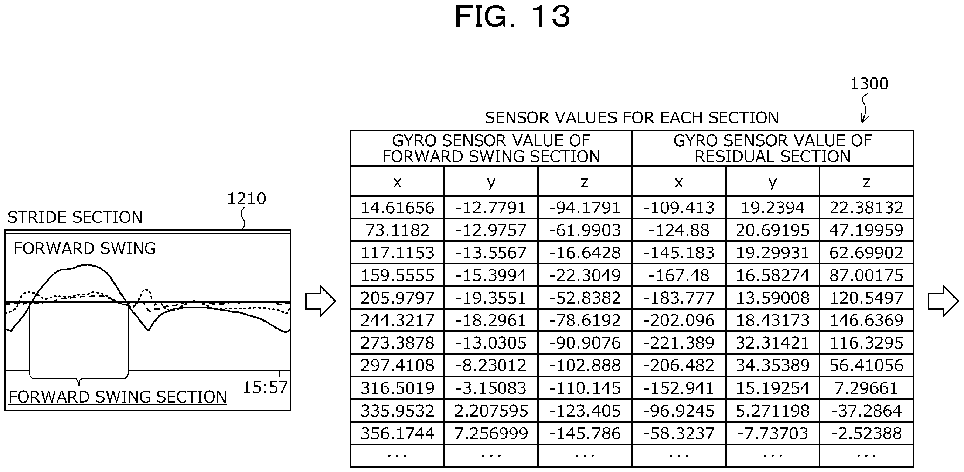

The information processing device 100 generates walking measurement data 1200 of the daily walking, which summarizes the received measured values, for the identified time span. The walking measurement data 1200 of the daily walking includes the measured values in each stride section 1210. The stride section 1210 includes the forward swing section. Next, description of FIG. 13 will be made.

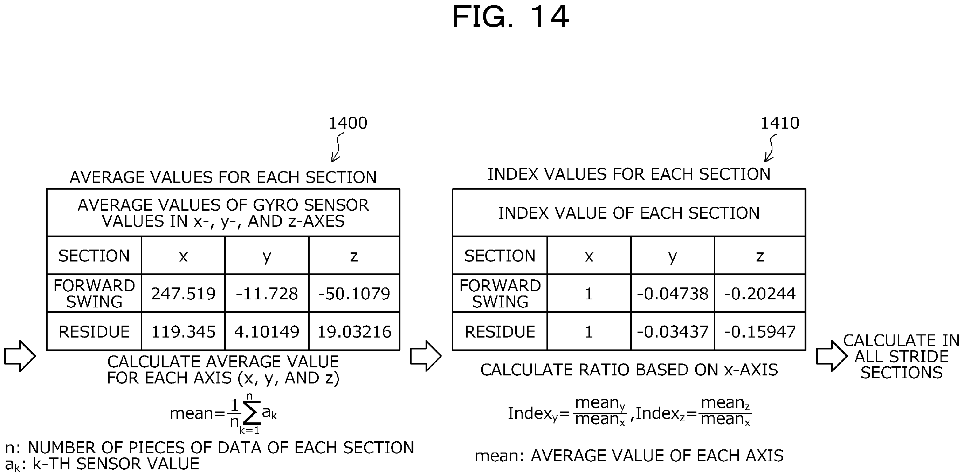

In FIG. 13, the information processing device 100 identifies a plurality of stride sections 1210 on the basis of the walking measurement data 1200 of the daily walking. As illustrated in table 1300, the information processing device 100 extracts the gyro sensor value of each axial direction among the x-, y-, and z-axis directions in the forward swing section for each stride section 1210, from the walking measurement data 1200 of the daily walking. As illustrated in table 1300, the information processing device 100 extracts the gyro sensor value of each axial direction among the x-, y-, and z-axis directions in other than the forward swing section for each stride section 1210, from the walking measurement data 1200 of the daily walking. Next, description of FIG. 14 will be made.

In FIG. 14, as illustrated in table 1400, the information processing device 100 calculates the average value of the gyro sensor values of each axial direction among the x-, y-, and z-axis directions in the forward swing section for each stride section 1210, on the basis of the extracted gyro sensor values, on the basis of above formula (1). As illustrated in table 1400, the information processing device 100 calculates the average value of the gyro sensor values of each axial direction among the x-, y-, and z-axis directions in other than the forward swing section for each stride section 1210, on the basis of the extracted gyro sensor values, on the basis of above formula (1).

As illustrated in table 1410, the information processing device 100 calculates the ratio of the average value of the gyro sensor values of each axial direction among the y- and z-axis directions to the average value of the gyro sensor values of the x-axis direction in the forward swing section for each stride section 1210. The information processing device 100 calculates the ratio of the average value of the gyro sensor values of each axial direction among the y- and z-axis directions to the average value of the gyro sensor values of the x-axis direction in the forward swing section for each stride section 1210, for example, on the basis of above formulas (2) and (3).

As illustrated in table 1410, the information processing device 100 calculates the ratio of the average value of the gyro sensor values of each axial direction among the y- and z-axis directions to the average value of the gyro sensor values of each axial direction among the y- and z-axis directions to the average value of the gyro sensor values of the x-axis direction in other than the forward swing section for each stride section 1210. The information processing device 100 calculates the ratio of the average value of the gyro sensor values of each axial direction among the y- and z-axis directions to the average value of the gyro sensor values of the x-axis direction in other than the forward swing section for each stride section 1210, for example, on the basis of above formulas (2) and (3).

This allows the information processing device 100 to calculate the index value indicating what kind of feature appears in the measured values in the stride section 1210 when the subject walks in a daily way. Therefore, the information processing device 100 may obtain information that serves as a guideline for identifying the stride section 1210 that is a section in which the subject walks normally, from among the plurality of stride sections 1210.

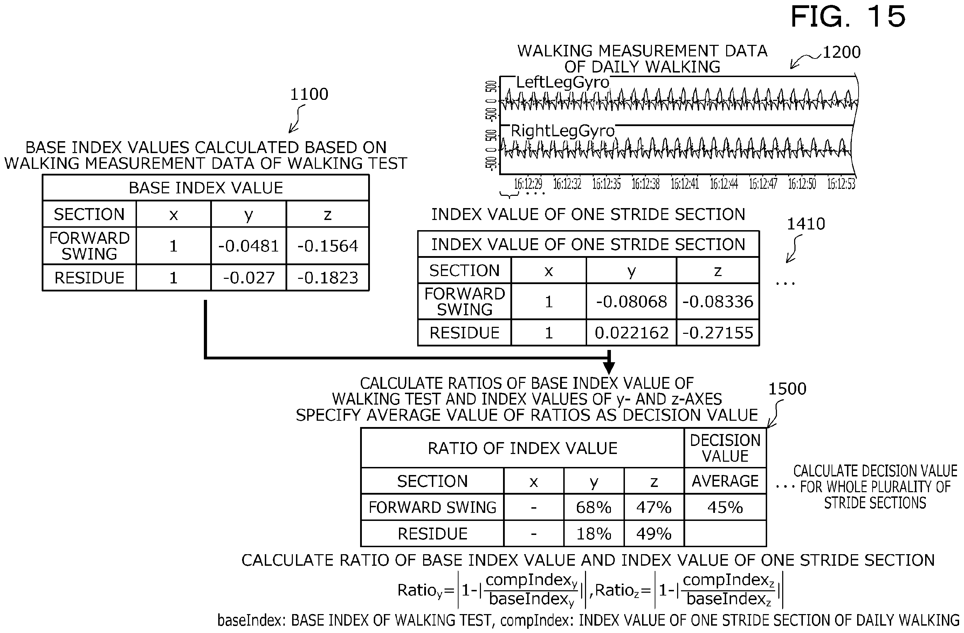

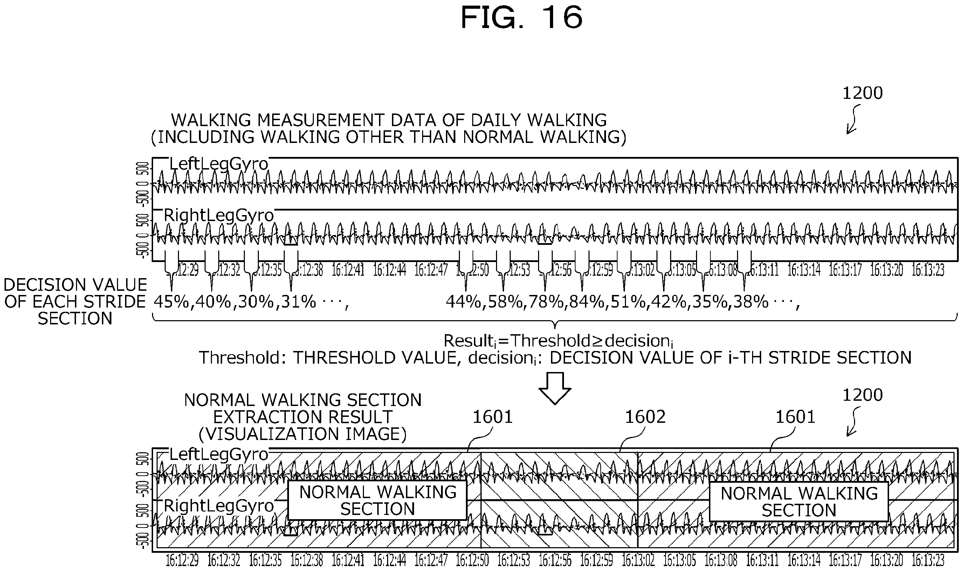

Next, an example in which the information processing device 100 extracts the normal walking section in which the subject walks normally will be described with reference to FIGs. 15 and 16.

FIGs. 15 and 16 are explanatory diagrams illustrating an example of extracting the normal walking section. In FIG. 15, the information processing device 100 calculates the ratio of the index value relating to the y-axis direction in the forward swing section of the stride section 1210 illustrated in table 1410 to the base index relating to the y-axis direction illustrated in table 1100, for each stride section 1210. In the example in FIG. 15, the information processing device 100 calculates the ratio = 68% on the basis of following formula (6), as illustrated in table 1500, for example.

The information processing device 100 calculates the ratio of the index value relating to the y-axis direction in other than the forward swing section of the stride section 1210 illustrated in table 1410 to the base index relating to the y-axis direction illustrated in table 1100, for each stride section 1210. In the example in FIG. 15, the information processing device 100 calculates the ratio = 18% on the basis of following formula (7), as illustrated in table 1500, for example.

The information processing device 100 calculates the ratio of the index value relating to the z-axis direction in the forward swing section of the stride section 1210 illustrated in table 1410 to the base index relating to the z-axis direction illustrated in table 1100, for each stride section 1210. In the example in FIG. 15, the information processing device 100 calculates the ratio = 47% on the basis of following formula (6), as illustrated in table 1500, for example.

The information processing device 100 calculates the ratio of the index value relating to the z-axis direction in other than the forward swing section of the stride section 1210 illustrated in table 1410 to the base index relating to the z-axis direction illustrated in table 1100, for each stride section 1210. In the example in FIG. 15, the information processing device 100 calculates the ratio = 49% on the basis of following formula (7), as illustrated in table 1500, for example.

$$Ratio_y = |1 - |compIndex_y/baseIndex_y|| \quad ... (6)$$

$$Ratio_z = |1 - |compIndex_z/baseIndex_z|| \quad ... (7)$$

The ratio of the index value relating to the y-axis direction to the base index relating to the y-axis direction is denoted by $Ratio_y$. The index value relating to the y-axis direction in the forward swing section or other than the forward swing section of the stride section 1210 is denoted by $compIndex_y$. The base index relating to the y-axis direction is denoted by $baseIndex_y$. The ratio of the index value relating to the z-axis direction to the base index relating to the z-axis direction is denoted by $Ratio_z$. The index value relating to the z-axis direction in the forward swing section or other than the forward swing section of the stride section 1210 is denoted by $compIndex_z$. The base index relating to the z-axis direction is denoted by $baseIndex_z$.

The information processing device 100 calculates the average value of the calculated ratios as a decision value decision. Next, description of FIG. 16 will be made.

In FIG. 16, the information processing device 100 determines whether or not the calculated decision value decision is equal to or lower than a threshold value Threshold, for each stride section 1210. The information processing device 100 determines whether or not the calculated decision value $decision_i$ is equal to or lower than the threshold value Threshold, for example, for the i-th stride section 1210.

For example, if the determination result $Result_i$ indicates the threshold value Threshold $\geq$ decision value $decision_i$, the information processing device 100 determines that the i-th stride section 1210 corresponds to the normal walking. On the other hand, for example, if the determination result $Result_i$ indicates the threshold value Threshold < decision value $decision_i$, the information processing device 100 determines that the i-th stride section 1210 does not correspond to the normal walking.

The information processing device 100 identifies a set of stride sections 1210 determined to correspond to the normal walking, as a normal walking section 1601. The information processing device 100 identifies a set of stride sections 1210 determined to not correspond to the normal walking, as a residual walking section 1602. The information processing device 100 may display, for example, the walking measurement data 1200 of the walking test, the identified normal walking section 1601, and the identified residual walking section 1602 on the display in association with each other.

This allows the information processing device 100 to identify the normal walking section in which the subject walks normally. Therefore, the information processing device 100 may identify, for example, the measured values in each axial direction in the normal walking section in which the subject walked normally, which are useful information when the health condition of the subject is analyzed. The information processing device 100 may facilitate the analysis of the health condition of the subject, for example, on the basis of the identified measured values.

(Setting Processing Procedure)

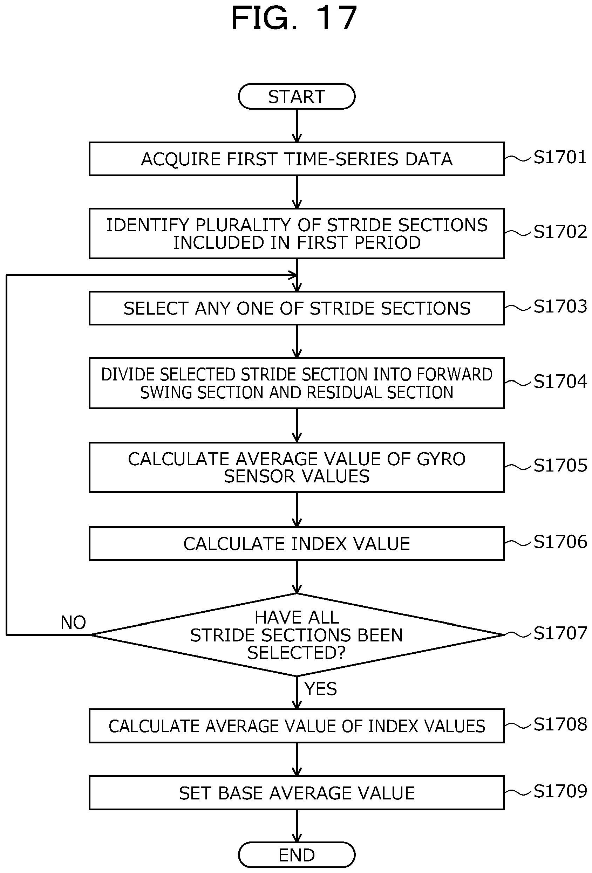

Next, an example of a setting processing procedure executed by the information processing device 100 will be described with reference to FIG. 17. The setting processing is implemented by, for example, the CPU 301, a storage area such as the memory 302 or the recording medium 305, and the network I/F 303 illustrated in FIG. 3.

FIG. 17 is a flowchart illustrating an example of the setting processing procedure. In FIG. 17, the information processing device 100 acquires the first time-series data including the measured values in the first period in which walking is performed normally (step S1701).

Next, the information processing device 100 identifies a plurality of stride sections included in the first period, on the basis of the acquired first time-series data (step S1702). Then, the information processing device 100 selects any one of the stride sections that has not yet been selected as a processing target, from among the plurality of stride sections (step S1703).

Next, the information processing device 100 divides the selected stride section into the forward swing section and a residual section (step S1704). Then, the information processing device 100 calculates the average value of the gyro sensor values in each section among the forward swing section and the residual section (step S1705).

Next, the information processing device 100 calculates the index value in each section among the forward swing section and the residual section, on the basis of the calculated average value (step S1706). Then, the information processing device 100 determines whether or not all the stride sections have been selected as processing targets (step S1707).

Here, when all the stride sections have been selected (step S1707: Yes), the information processing device 100 proceeds to the process in step S1708. On the other hand, when there is an unselected stride section (step S1707: No), the information processing device 100 returns to the process in step S1703.

In step S1708, the information processing device 100 calculates the average value of the index values in each section among the forward swing section and the residual section for the whole plurality of stride sections (step S1708).

Next, the information processing device 100 sets the calculated average value for each section among the forward swing section and the residual section, as a base average value in each section (step S1709). Then, the information processing device 100 ends the setting processing. This allows the information processing device 100 to calculate the base index value indicating what kind of feature appears in the measured values in the stride section when the subject walks normally. Therefore, the information processing device 100 may obtain, from the walking measurement data when the subject walks in a daily way, information that serves as a guideline for identifying a stride section that is a section in which the subject walks normally, in a period in which the subject walks in a daily way.

(Determination Processing Procedure)

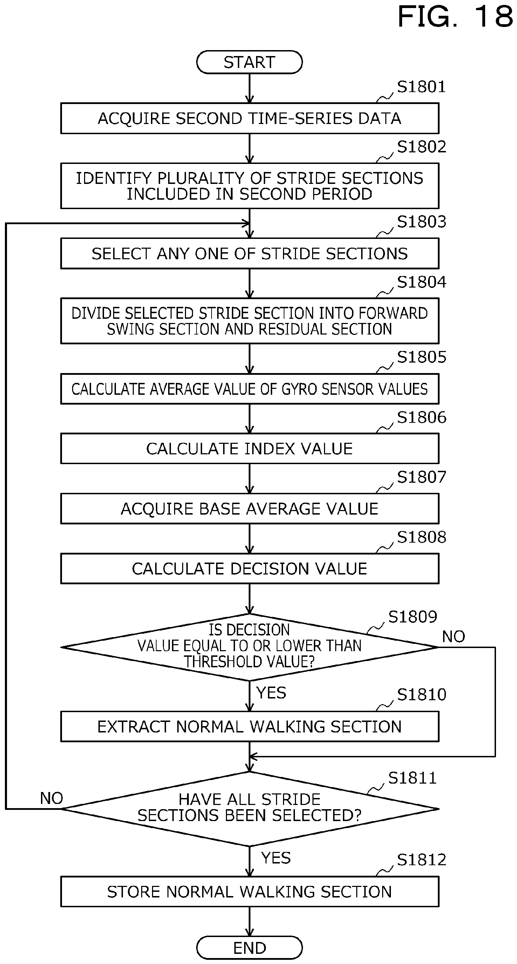

Next, an example of a determination processing procedure executed by the information processing device 100 will be described with reference to FIG. 18. The determination processing is implemented by, for example, the CPU 301, a storage area such as the memory 302 or the recording medium 305, and the network I/F 303 illustrated in FIG. 3.

FIG. 18 is a flowchart illustrating an example of the determination processing procedure. The information processing device 100 acquires the second time-series data including the measured values in the second period in which walking is performed in a daily way (step S1801).

Next, the information processing device 100 identifies a plurality of stride sections included in the second period, on the basis of the acquired second time-series data (step S1802). Then, the information processing device 100 selects any one of the stride sections that has not yet been selected as a processing target, from among the plurality of stride sections (step S1803).

Next, the information processing device 100 divides the selected stride section into the forward swing section and a residual section (step S1804). Then, the information processing device 100 calculates the average value of the gyro sensor values in each section among the forward swing section and the residual section (step S1805).

Next, the information processing device 100 calculates the index value in each section among the forward swing section and the residual section, on the basis of the calculated average value (step S1806). Then, the information processing device 100 acquires the base average value in each section among the forward swing section and the residual section (step S1807).

Next, the information processing device 100 calculates the decision value by comparing the calculated index value and the acquired base average value for each section among the forward swing section and the residual section (step S1808). Then, the information processing device 100 determines whether or not the calculated decision value is equal to or lower than the threshold value (step S1809).

Here, when the calculated decision value is equal to or lower than the threshold value (step S1809: Yes), the information processing device 100 extracts the selected stride section as a normal walking section (step S1810) and proceeds to the process in step S1811. On the other hand, when the calculated decision value is higher than the threshold value (step S1809: No), the information processing device 100 proceeds to the process in step S1811.

In step S1811, the information processing device 100 determines whether or not all the stride sections have been selected as processing targets (step S1811). Here, when all the stride sections have been selected (step S1811: Yes), the information processing device 100 proceeds to the process in step S1812. On the other hand, when there is an unselected stride section (step S1811: No), the information processing device 100 returns to the process in step S1803.

In step S1812, the information processing device 100 stores the extracted normal walking section (step S1812). Then, the information processing device 100 ends the determination processing. This allows the information processing device 100 to identify the normal walking section in which the subject walks normally. Therefore, the information processing device 100 may identify, for example, the measured values in each axial direction in the normal walking section in which the subject walked normally, which are useful information when the health condition of the subject is analyzed. The information processing device 100 may facilitate the analysis of the health condition of the subject, for example, on the basis of the identified measured values.

As described above, according to the information processing device 100, the first time-series data including the measured values regarding the movement of the legs of the subject in the first period in which the subject has traveled in a predetermined walking manner, in each axial direction among the plurality of axial directions may be acquired. According to the information processing device 100, on the basis of the acquired first time-series data, the first index value may be calculated in the first period for the first section in which the subject swings one leg, for each first stride section corresponding to one step of the subject. According to the information processing device 100, the second time-series data including the measured values regarding the movement of the legs of the subject in the second period different from the first period, in each axial direction may be acquired. According to the information processing device 100, on the basis of the acquired second time-series data, the second index value may be calculated in the second period for the second section in which the subject swings one leg, for each second stride section corresponding to one step of the subject. According to the information processing device 100, it may be determined whether or not each of the second stride sections in the second period corresponds to the predetermined walking manner, on the basis of the calculated first index value and the calculated second index value. This allows the information processing device 100 to identify the second stride section corresponding to the predetermined walking manner and to facilitate the analysis of the health condition of the subject.

According to the information processing device 100, the third index value may be calculated in the first period for the third section other than the first section, for each first stride section, on the basis of the acquired first time-series data. According to the information processing device 100, the fourth index value may be calculated in the second period for the fourth section other than the second section, for each second stride section, on the basis of the acquired second time-series data. According to the information processing device 100, it may be determined whether or not each of the second stride sections in the second period corresponds to the predetermined walking manner, on the basis of the calculated first index value, the calculated second index value, the calculated third index value, and the calculated fourth index value. This allows the information processing device 100 to improve the accuracy of determining whether or not the correspondence to the predetermined walking manner is applicable.

According to the information processing device 100, the ratio of the statistical value of the measured values in one axial direction to the statistical value of the measured values in another axial direction may be calculated in the first period for the first section for each first stride section, as the first index value. According to the information processing device 100, the ratio of the statistical value of the measured values in one axial direction to the statistical value of the measured values in another axial direction may be calculated in the second period for the second section for each second stride section as the second index value. According to the information processing device 100, the ratio of the statistical value of the measured values in one axial direction to the statistical value of the measured values in another axial direction may be calculated in the first period for the third section for each first stride section, as the third index value. According to the information processing device 100, the ratio of the statistical value of the measured values in one axial direction to the statistical value of the measured values in another axial direction may be calculated in the second period for the fourth section for each second stride section, as the fourth index value. This allows the information processing device 100 to accurately calculate the first index value, the second index value, the third index value, and the fourth index value.

According to the information processing device 100, a statistical value of the percentage of the second index value to the first index value and the percentage of the fourth index value to the third index value may be calculated in the second period for each of the second stride sections. According to the information processing device 100, when the calculated statistical value is equal to or lower than a predetermined threshold value, it may be determined that the second stride section corresponds to the predetermined walking manner. This allows the information processing device 100 to improve the accuracy of determining whether or not the correspondence to the predetermined walking manner is applicable.

According to the information processing device 100, the first time-series data including the angular velocity regarding the movement of the legs of the subject in the first period in each axial direction may be acquired. According to the information processing device 100, the second time-series data including the angular velocity regarding the movement of the legs of the subject in the second period in each axial direction may be acquired. This allows the information processing device 100 to acquire the first time-series data and the second time-series data including the measured values for which it is deemed that the feature of the predetermined walking manner is likely to appear. Therefore, the information processing device 100 may improve the accuracy of determining whether or not the correspondence to the predetermined walking manner is applicable.

According to the information processing device 100, a plurality of axial directions including the front-back direction, the left-right direction, and the up-down direction of the subject may be set. This allows the information processing device 100 to acquire the first time-series data and the second time-series data including the measured values for which it is deemed that the feature of the predetermined walking manner is likely to appear. Therefore, the information processing device 100 may improve the accuracy of determining whether or not the correspondence to the predetermined walking manner is applicable.

According to the information processing device 100, the first time-series data including the measured values regarding the movement of the legs of the subject in the first period in which the subject has traveled in a predetermined walking manner, in each axial direction among the plurality of axial directions may be acquired. According to the information processing device 100, on the basis of the acquired first time-series data, the first index value may be calculated in the first period for a residual section other than the first section in which the subject swings one leg, for each first stride section corresponding to one step of the subject. According to the information processing device 100, the second time-series data including the measured values regarding the movement of the legs of the subject in the second period different from the first period, in each axial direction may be acquired. According to the information processing device 100, on the basis of the acquired second time-series data, the second index value may be calculated in the second period for a residual section other than the second section in which the subject swings one leg, for each second stride section corresponding to one step of the subject. According to the information processing device 100, it may be determined whether or not each of the second stride sections in the second period corresponds to the walking manner, on the basis of the calculated first index value and the calculated second index value. This allows the information processing device 100 to identify the second stride section corresponding to the predetermined walking manner and to facilitate the analysis of the health condition of the subject.

Note that the information processing method described in the present embodiment may be implemented by executing a program prepared in advance, on a computer such as a personal computer (PC) or a workstation. The information processing program described in the present embodiment is executed by being recorded on a computer-readable recording medium and being read from the recording medium by the computer. The recording medium is a hard disk, a flexible disk, a compact disc (CD)-ROM, a magneto-optical disc (MO), a digital versatile disc (DVD), or the like. In addition, the information processing program described in the present embodiment may be distributed via a network such as the Internet.

**Claims**

1. An information processing program that causes a computer to execute a process comprising:

   acquiring first time-series data that includes measured values regarding movement of legs of a subject in a first period in which the subject travels in a walking manner for each of a plurality of axial directions;
   acquiring a first index value that relates to value of the measured values of one axial direction of the plurality of axial directions over value of the measured values of another axial direction of the plurality of axial directions for a first section in which the subject swings one of the legs for each of first stride sections that corresponds to one step of the subject in the first period based on the acquired first time-series data;

acquiring second time-series data that includes second measured values regarding the movement of the legs of the subject in a second period different from the first period in which the subject travels in the walking manner for each of the plurality of axial directions;

acquiring a second index value that relates to value of the measured values of one axial direction of the plurality of axial directions over value of the measured values of another axial direction of the plurality of axial directions for a second section in which the subject swings one of the legs for each of second stride sections that corresponds to one step of the subject in the second period based on the acquired second time-series data; and

determining whether or not each of the second stride sections in the second period corresponds to the walking manner, based on the acquired first index value and the acquired second index value, wherein, when a percentage of the second index value for any one of the second stride sections to the first index value is equal to or lower than a predetermined threshold value, it is determined that the any one of the second stride sections corresponds to the walking manner.

2. The information processing program according to claim 1, wherein the process further comprising:

acquiring a third index value that relates to value of the measured values of one axial direction of the plurality of axial directions over value of the measured values of another axial direction of the plurality of axial directions for a third section other than the first section in which the subject swings one of the legs for each of third stride sections that corresponds to one step of the subject in the first period based on the acquired first time-series data; and

acquiring a fourth index value that relates to value of the measured values of one axial direction of the plurality of axial directions over value of the measured values of another axial direction of the plurality of axial directions for a fourth section other than the second section in which the subject swings one of the legs for each of third stride sections that corresponds to one step of the subject in the second period based on the acquired second time-series data, wherein

the determining includes determining whether or not each of the second stride sections in the second period corresponds to the walking manner based on the acquired first index value, the acquired second index value, the acquired third index value, and the acquired fourth index value.

3. The information processing program according to claim 2, wherein

the acquiring the first index value includes acquiring, as the first index value, a ratio of a statistical value of the measured values in the one axial direction to the statistical value of the measured values in the another axial direction among the plurality of axial directions, in the first period for the first section for each of the first stride sections,

the acquiring the second index value includes acquiring, as the second index value, the ratio of the statistical value of the measured values in the one axial direction to the statistical value of the measured values in the another axial direction among the plurality of axial directions, in the second period for the second section for each of the second stride sections,

the acquiring the third index value includes acquiring, as the third index value, the ratio of the statistical value of the measured values in the one axial direction to the statistical value of the measured value in the another axial direction among the plurality of axial directions, in the first period for the third section for each of the first stride sections, and

the acquiring the fourth index value includes acquiring, as the fourth index value, the ratio of the statistical values of the measured values in the one axial direction to the statistical value of the measured values in the another axial direction among the plurality of axial directions, in the second period for the fourth section for each of the second stride sections.

4. The information processing program according to claim 2 or 3, wherein the determining whether or not each of the second stride sections in the second period corresponds to the walking manner is based on the first index value, the second index value, the third index value and the fourth index value, and wherein the determination that the any one of the second stride sections corresponds to the walking manner, when the percentage of the second index value for the any one of the second stride sections to the first index value is equal to or lower than the predetermined threshold value, is when the percentage of the second index value for the any one of the second stride sections to the first index value is equal to or lower than the predetermined threshold value and a percentage of the fourth index value for the any one of the second stride sections to the third index value is equal to or lower than a second predetermined threshold value.

5. The information processing program according to any one of claims 1 to 4, wherein

> the first time-series data includes angular velocity regarding the movement of the legs of the subject in the first period in the each axial direction, and
> the second time-series data includes the angular velocity regarding the movement of the legs of the subject in the second period in the each axial direction.

6. The information processing program according to any one of claims 1 to 5, wherein the plurality of axial directions includes a front-back direction, a left-right direction, and an up-down direction of the subject.

7. An information processing method for a computer to execute a process comprising:

> acquiring first time-series data that includes measured values regarding movement of legs of a subject in a first period in which the subject travels in a walking manner for each of a plurality of axial directions;
> acquiring a first index value that relates to value of the measured values of one axial direction of the plurality of axial directions over value of the measured values of another axial direction of the plurality of axial directions for a first section in which the subject swings one of the legs for each of first stride sections that corresponds to one step of the subject in the first period based on the acquired first time-series data;
> acquiring second time-series data that includes second measured values regarding the movement of the legs of the subject in a second period different from the first period in which the subject travels in the walking manner for each of the plurality of axial directions;
> acquiring a second index value that relates to value of the measured values of one axial direction of the plurality of axial directions over value of the measured values of another axial direction of the plurality of axial directions for a second section in which the subject swings one of the legs for each of second stride sections that corresponds to one step of the subject in the second period based on the acquired second time-series data; and
> determining whether or not each of the second stride sections in the second period corresponds to the walking manner, based on the acquired first index value and the acquired second index value, wherein, when a percentage of the second index value for any one of the second stride sections to the first index value is equal to or lower than a predetermined threshold value, it is determined that the any one of the second stride sections corresponds to the walking manner.

8. An information processing device comprising: a control unit configured to:

> acquire first time-series data that includes measured values regarding movement of legs of a subject in a first period in which the subject travels in a walking manner for each of a plurality of axial directions,
> acquire a first index value that relates to value of the measured values of one axial direction of the plurality of axial directions over value of the measured values of another axial direction of the plurality of axial directions for a first section in which the subject swings one of the legs for each of first stride sections that corresponds to one step of the subject in the first period based on the acquired first time-series data,
> acquire second time-series data that includes second measured values regarding the movement of the legs of the subject in a second period different from the first period in which the subject travels in the walking manner for each of the plurality of axial directions,
> acquire a second index value that relates to value of the measured values of one axial direction of the plurality of axial directions over value of the measured values of another axial direction of the plurality of axial directions for a second section in which the subject swings one of the legs for each of second stride sections that corresponds to one step of the subject in the second period based on the acquired second time-series data, and
> determine whether or not each of the second stride sections in the second period corresponds to the walking manner, based on the acquired first index value and the acquired second index value, wherein, when a percentage of the second index value for any one of the second stride sections to the first index value is equal to or lower than a predetermined threshold value, it is determined that the any one of the second stride sections corresponds to the walking manner.

**Patentansprüche**

1. Informationsverarbeitungsprogramm, das einen Computer veranlasst, einen Prozess auszuführen, der Folgendes umfasst:

> Erfassen von ersten Zeitreihendaten, die gemessene Werte bezüglich einer Bewegung von Beinen eines Subjekts in einer ersten Periode einschließen, in der sich das Subjekt in einer Gehweise für jede einer Vielzahl von axialen Richtungen bewegt;

Erfassen eines ersten Indexwertes, der sich auf einen Wert der gemessenen Werte einer axialen Richtung der Vielzahl von axialen Richtungen gegenüber einem Wert der gemessenen Werte einer anderen axialen Richtung der Vielzahl von axialen Richtungen bezieht, für einen ersten Abschnitt, in dem das Subjekt eines der Beine für jeden von ersten Schrittabschnitten schwingt, der einem Schritt des Subjekts in der ersten Periode entspricht, basierend auf den erfassten ersten Zeitreihendaten;

Erfassen von zweiten Zeitreihendaten, die zweite gemessene Werte bezüglich der Bewegung der Beine des Subjekts in einer zweiten Periode einschließen, die sich von der ersten Periode unterscheidet, in der sich das Subjekt in der Gehweise für jede der Vielzahl von axialen Richtungen bewegt;

Erfassen eines zweiten Indexwertes, der sich auf einen Wert der gemessenen Werte einer axialen Richtung der Vielzahl von axialen Richtungen gegenüber einem Wert der gemessenen Werte einer anderen axialen Richtung der Vielzahl von axialen Richtungen bezieht, für einen zweiten Abschnitt, in dem das Subjekt eines der Beine für jeden von zweiten Schrittabschnitten schwingt, der einem Schritt des Subjekts in der zweiten Periode entspricht, basierend auf den erfassten zweiten Zeitreihendaten; und

Bestimmen, ob jeder der zweiten Schrittabschnitte in der zweiten Periode der Gehweise entspricht oder nicht, basierend auf dem erfassten ersten Indexwert und dem erfassten zweiten Indexwert, wobei, wenn ein Prozentsatz des zweiten Indexwertes für beliebigen der zweiten Schrittabschnitte zu dem ersten Indexwert gleich oder niedriger als ein vorbestimmter Schwellenwert ist, bestimmt wird, dass der beliebige der zweiten Schrittabschnitte der Gehweise entspricht.

2. Informationsverarbeitungsprogramm nach Anspruch 1, wobei der Prozess weiter Folgendes umfasst:

Erfassen eines dritten Indexwertes, der sich auf einen Wert der gemessenen Werte einer axialen Richtung der Vielzahl von axialen Richtungen gegenüber einem Wert der gemessenen Werte einer anderen axialen Richtung der Vielzahl von axialen Richtungen bezieht, für einen dritten Abschnitt, der nicht der erste Abschnitt ist, in dem das Subjekt eines der Beine für jeden von dritten Schrittabschnitten schwingt, der einem Schritt des Subjekts in der ersten Periode entspricht, basierend auf den erfassten ersten Zeitreihendaten; und

Erfassen eines vierten Indexwertes, der sich auf einen Wert der gemessenen Werte einer axialen Richtung der Vielzahl von axialen Richtungen gegenüber einem Wert der gemessenen Werte einer anderen axialen Richtung der Vielzahl von axialen Richtungen für einen vierten Abschnitt bezieht, der nicht der zweite Abschnitt ist, in dem das Subjekt eines der Beine für jeden von dritten Schrittabschnitten schwingt, der einem Schritt des Subjekts in der zweiten Periode entspricht, basierend auf den erfassten zweiten Zeitreihendaten, wobei

das Bestimmen das Bestimmen einschließt, ob jeder der zweiten Schrittabschnitte in der zweiten Periode der Gehweise entspricht oder nicht, basierend auf dem erfassten ersten Indexwert, dem erfassten zweiten Indexwert, dem erfassten dritten Indexwert und dem erfassten vierten Indexwert.

3. Informationsverarbeitungsprogramm nach Anspruch 2, wobei

das Erfassen des ersten Indexwertes das Erfassen eines Verhältnisses eines statistischen Wertes der gemessenen Werte in der einen axialen Richtung zu dem statistischen Wert der gemessenen Werte in der anderen axialen Richtung unter der Vielzahl von axialen Richtungen in der ersten Periode für den ersten Abschnitt für jeden der ersten Schrittabschnitte als den ersten Indexwert einschließt,

das Erfassen des zweiten Indexwertes das Erfassen des Verhältnisses des statistischen Wertes der gemessenen Werte in der einen axialen Richtung zu dem statistischen Wert der gemessenen Werte in der anderen axialen Richtung unter der Vielzahl von axialen Richtungen in der zweiten Periode für den zweiten Abschnitt für jeden der zweiten Schrittabschnitte als den zweiten Indexwert einschließt,

das Erfassen des dritten Indexwertes das Erfassen des Verhältnisses des statistischen Wertes der gemessenen Werte in der einen axialen Richtung zu dem statistischen Wert des gemessenen Wertes in der anderen axialen Richtung unter der Vielzahl von axialen Richtungen in der ersten Periode für den dritten Abschnitt für jeden der ersten Schrittabschnitte als den dritten Indexwert einschließt, und

das Erfassen des vierten Indexwertes das Erfassen des Verhältnisses der statistischen Werte der gemessenen Werte in der einen axialen Richtung zu dem statistischen Wert der gemessenen Werte in der anderen axialen Richtung unter der Vielzahl von axialen Richtungen in der zweiten Periode für den vierten Abschnitt für jeden der zweiten Schrittabschnitte als den vierten Indexwert einschließt.

**4.** Informationsverarbeitungsprogramm nach Anspruch 2 oder 3, wobei das Bestimmen, ob jeder der zweiten Schrittabschnitte in der zweiten Periode der Gehweise entspricht oder nicht, auf dem ersten Indexwert, dem zweiten Indexwert, dem dritten Indexwert und dem vierten Indexwert basiert, und wobei das Bestimmen, dass der beliebige eine der zweiten Schrittabschnitte der Gehweise entspricht, wenn der Prozentsatz des zweiten Indexwertes für den beliebigen einen der zweiten Schrittabschnitte zu dem ersten Indexwert gleich oder niedriger als der vorbestimmte Schwellenwert ist, ist, wenn der Prozentsatz des zweiten Indexwertes für den beliebigen einen der zweiten Schrittabschnitte zu dem ersten Indexwert gleich oder niedriger als der vorbestimmte Schwellenwert ist und ein Prozentsatz des vierten Indexwertes für den beliebigen einen der zweiten Schrittabschnitte zu dem dritten Indexwert gleich oder niedriger als ein zweiter vorbestimmter Schwellenwert ist.

**5.** Informationsverarbeitungsprogramm nach einem der Ansprüche 1 bis 4, wobei

die ersten Zeitreihendaten die Winkelgeschwindigkeit bezüglich der Bewegung der Beine des Subjekts in der ersten Periode in jeder axialen Richtung einschließen, und
die zweiten Zeitreihendaten die Winkelgeschwindigkeit bezüglich der Bewegung der Beine des Subjekts in der zweiten Periode in der jeweiligen axialen Richtung einschließen.

**6.** Informationsverarbeitungsprogramm nach einem der Ansprüche 1 bis 5, wobei die Vielzahl von axialen Richtungen eine Vorwärts-Rückwärts-Richtung, eine Links-Rechts-Richtung und eine Aufwärts-Abwärts-Richtung des Subjekts einschließt.

**7.** Informationsverarbeitungsverfahren für einen Computer zum Ausführen eines Prozesses, der Folgendes umfasst:

Erfassen von ersten Zeitreihendaten, die gemessene Werte bezüglich einer Bewegung von Beinen eines Subjekts in einer ersten Periode einschließen, in der sich das Subjekt in einer Gehweise für jede einer Vielzahl von axialen Richtungen bewegt;
Erfassen eines ersten Indexwertes, der sich auf einen Wert der gemessenen Werte einer axialen Richtung der Vielzahl von axialen Richtungen gegenüber einem Wert der gemessenen Werte einer anderen axialen Richtung der Vielzahl von axialen Richtungen bezieht, für einen ersten Abschnitt, in dem das Subjekt eines der Beine für jeden von ersten Schrittabschnitten schwingt, der einem Schritt des Subjekts in der ersten Periode entspricht, basierend auf den erfassten ersten Zeitreihendaten;
Erfassen von zweiten Zeitreihendaten, die zweite gemessene Werte bezüglich der Bewegung der Beine des Subjekts in einer zweiten Periode einschließen, die sich von der ersten Periode unterscheidet, in der sich das Subjekt in der Gehweise für jede der Vielzahl von axialen Richtungen bewegt;
Erfassen eines zweiten Indexwertes, der sich auf einen Wert der gemessenen Werte einer axialen Richtung der Vielzahl von axialen Richtungen gegenüber einem Wert der gemessenen Werte einer anderen axialen Richtung der Vielzahl von axialen Richtungen bezieht, für einen zweiten Abschnitt, in dem das Subjekt eines der Beine für jeden von zweiten Schrittabschnitten schwingt, der einem Schritt des Subjekts in der zweiten Periode entspricht, basierend auf den erfassten zweiten Zeitreihendaten; und
Bestimmen, ob jeder der zweiten Schrittabschnitte in der zweiten Periode der Gehweise entspricht oder nicht, basierend auf dem erfassten ersten Indexwert und dem erfassten zweiten Indexwert, wobei, wenn ein Prozentsatz des zweiten Indexwertes für einen beliebigen der zweiten Schrittabschnitte zu dem ersten Indexwert gleich oder niedriger als ein vorbestimmter Schwellenwert ist, bestimmt wird, dass der beliebige der zweiten Schrittabschnitte der Gehweise entspricht.

**8.** Informationsverarbeitungsvorrichtung, die Folgendes umfasst:
eine Steuereinheit, konfiguriert zum:

Erfassen von ersten Zeitreihendaten, die gemessene Werte bezüglich einer Bewegung von Beinen eines Subjekts in einer ersten Periode einschließen, in der sich das Subjekt in einer Gehweise für jede einer Vielzahl von axialen Richtungen bewegt,
Erfassen eines ersten Indexwertes, der sich auf einen Wert der gemessenen Werte einer axialen Richtung der Vielzahl von axialen Richtungen gegenüber einem Wert der gemessenen Werte einer anderen axialen Richtung der Vielzahl von axialen Richtungen bezieht, für einen ersten Abschnitt, in dem das Subjekt eines der Beine für jeden von ersten Schrittabschnitten schwingt, der einem Schritt des Subjekts in der ersten Periode entspricht, basierend auf den erfassten ersten Zeitreihendaten,

Erfassen von zweiten Zeitreihendaten, die zweite gemessene Werte bezüglich der Bewegung der Beine des Subjekts in einer zweiten Periode einschließen, die sich von der ersten Periode unterscheidet, in der sich das Subjekt in der Gehweise für jede der Vielzahl von axialen Richtungen bewegt,

Erfassen eines zweiten Indexwertes, der sich auf einen Wert der gemessenen Werte einer axialen Richtung der Vielzahl von axialen Richtungen gegenüber einem Wert der gemessenen Werte einer anderen axialen Richtung der Vielzahl von axialen Richtungen bezieht, für einen zweiten Abschnitt, in dem das Subjekt eines der Beine für jeden von zweiten Schrittabschnitten schwingt, der einem Schritt des Subjekts in der zweiten Periode entspricht, basierend auf den erfassten zweiten Zeitreihendaten, und

Bestimmen, ob jeder der zweiten Schrittabschnitte in der zweiten Periode der Gehweise entspricht oder nicht, basierend auf dem erfassten ersten Indexwert und dem erfassten zweiten Indexwert, wobei, wenn ein Prozentsatz des zweiten Indexwertes für einen beliebigen der zweiten Schrittabschnitte zu dem ersten Indexwert gleich oder niedriger als ein vorbestimmter Schwellenwert ist, bestimmt wird, dass der beliebige der zweiten Schrittabschnitte der Gehweise entspricht.

## Revendications

1. Programme de traitement d'informations qui amène un ordinateur à mettre en oeuvre un processus comprenant :

l'acquisition de premières données chronologiques qui incluent des valeurs mesurées concernant un déplacement de jambes d'un sujet dans une première période dans laquelle le sujet se déplace en marchant pour chacune d'une pluralité de directions axiales ;

l'acquisition d'une première valeur d'indice qui se rapporte à une valeur des valeurs mesurées d'une direction axiale de la pluralité de directions axiales sur une valeur des valeurs mesurées d'une autre direction axiale de la pluralité de directions axiales pour une première section dans laquelle le sujet balance l'une des jambes pour chacune de premières sections d'enjambée qui correspond à un pas du sujet dans la première période sur la base des premières données chronologiques acquises ;

l'acquisition de deuxièmes données chronologiques qui incluent des deuxièmes valeurs mesurées concernant le déplacement des jambes du sujet dans une deuxième période différente de la première période dans laquelle le sujet se déplace en marchant pour chacune de la pluralité de directions axiales ;

l'acquisition d'une deuxième valeur d'indice qui se rapporte à une valeur des valeurs mesurées d'une direction axiale de la pluralité de directions axiales sur une valeur des valeurs mesurées d'une autre direction axiale de la pluralité de directions axiales pour une deuxième section dans laquelle le sujet balance l'une des jambes pour chacune de deuxièmes sections d'enjambée qui correspond à un pas du sujet dans la deuxième période sur la base des deuxièmes données chronologiques acquises ; et

le fait de déterminer si chacune des deuxièmes sections d'enjambée dans la deuxième période correspond ou non à la manière de marcher, sur la base de la première valeur d'indice acquise et de la deuxième valeur d'indice acquise, dans lequel, lorsqu'un pourcentage de la deuxième valeur d'indice pour l'une quelconque des deuxièmes sections d'enjambée par rapport à la première valeur d'indice est inférieur ou égal à une valeur seuil prédéterminée, il est déterminé que la quelconque des deuxièmes sections d'enjambée correspond à la manière de marcher.

2. Programme de traitement d'informations selon la revendication 1, dans lequel le processus comprend en outre :

l'acquisition d'une troisième valeur d'indice qui se rapporte à une valeur des valeurs mesurées d'une direction axiale de la pluralité de directions axiales sur une valeur des valeurs mesurées d'une autre direction axiale de la pluralité de directions axiales pour une troisième section autre que la première section dans laquelle le sujet balance l'une des jambes pour chacune de troisièmes sections d'enjambée qui correspond à un pas du sujet dans la première période sur la base des premières données chronologiques acquises ; et

l'acquisition d'une quatrième valeur d'indice qui se rapporte à une valeur des valeurs mesurées d'une direction axiale de la pluralité de directions axiales sur une valeur des valeurs mesurées d'une autre direction axiale de la pluralité de directions axiales pour une quatrième section autre que la deuxième section dans laquelle le sujet balance l'une des jambes pour chacune de troisièmes sections d'enjambée qui correspond à un pas du sujet dans la deuxième période sur la base des deuxièmes données chronologiques acquises, dans lequel la détermination inclut le fait de déterminer si chacune des deuxièmes sections d'enjambée dans la deuxième période correspond ou non à la manière de marcher sur la base de la première valeur d'indice acquise, de la deuxième valeur d'indice acquise, de la troisième valeur d'indice acquise et de la quatrième valeur d'indice acquise.

3. Programme de traitement d'informations selon la revendication 2, dans lequel

l'acquisition de la première valeur d'indice inclut l'acquisition, en tant que première valeur d'indice, d'un rapport d'une valeur statistique des valeurs mesurées dans la une direction axiale sur la valeur statistique des valeurs mesurées dans l'autre direction axiale parmi la pluralité de directions axiales, dans la première période pour la première section pour chacune des premières périodes d'enjambée,

l'acquisition de la deuxième valeur d'indice inclut l'acquisition, en tant que deuxième valeur d'indice, du rapport de la valeur statistique des valeurs mesurées dans la une direction axiale sur la valeur statistique des valeurs mesurées dans l'autre direction axiale parmi la pluralité de directions axiales, dans la deuxième période pour la deuxième section pour chacune des deuxièmes périodes d'enjambée,

l'acquisition de la troisième valeur d'indice inclut l'acquisition, en tant que troisième valeur d'indice, du rapport de la valeur statistique des valeurs mesurées dans la une direction axiale sur la valeur statistique de la valeur mesurée dans l'autre direction axiale parmi la pluralité de directions axiales, dans la première période pour la troisième section pour chacune des premières périodes d'enjambée, et

l'acquisition de la quatrième valeur d'indice inclut l'acquisition, en tant que quatrième valeur d'indice, du rapport des valeurs statistiques des valeurs mesurées dans la une direction axiale sur la valeur statistique des valeurs mesurées dans l'autre direction axiale parmi la pluralité de directions axiales, dans la deuxième période pour la quatrième section pour chacune des deuxièmes périodes d'enjambée.

4. Programme de traitement d'informations selon la revendication 2 ou la revendication 3, dans lequel le fait de déterminer si chacune des deuxièmes sections d'enjambée dans la deuxième période correspond ou non à la manière de marcher est basé sur la première valeur d'indice, la deuxième valeur d'indic, la troisième valeur d'indice et la quatrième valeur d'indice, et dans lequel la détermination que la quelconque des deuxièmes sections d'enjambée correspond à la manière de marcher, lorsque le pourcentage de la deuxième valeur d'indice pour la quelconque des deuxièmes sections d'enjambée par rapport à la première valeur d'indice est inférieur ou égal à la valeur seuil prédéterminée, est lorsque le pourcentage de la deuxième valeur d'indice pour la quelconque des deuxièmes sections d'enjambée par rapport à la première valeur d'indice est inférieur ou égal à la valeur seuil prédéterminée et un pourcentage de la quatrième valeur d'indice pour la quelconque des deuxièmes sections d'enjambée par rapport à la troisième valeur d'indice est inférieur ou égal à une deuxième valeur seuil prédéterminée.

5. Programme de traitement d'informations selon l'une quelconque des revendications 1 à 4, dans lequel

les premières données chronologiques incluent la vitesse angulaire concernant le déplacement des jambes du sujet dans la première période dans chaque direction axiale, et les deuxièmes données chronologiques incluent la vitesse angulaire concernant le déplacement des jambes du sujet dans la deuxième période dans chaque direction axiale.

6. Programme de traitement d'informations selon l'une quelconque des revendications 1 à 5, dans lequel la pluralité de directions axiales incluent une direction avant-arrière, une direction gauche-droite et une direction haut-bas du sujet.

7. Procédé de traitement d'informations pour qu'un ordinateur mette en oeuvre un processus comprenant :

l'acquisition de premières données chronologiques qui incluent des valeurs mesurées concernant un déplacement de jambes d'un sujet dans une première période dans laquelle le sujet se déplace en marchant pour chacune d'une pluralité de directions axiales ;

l'acquisition d'une première valeur d'indice qui se rapporte à une valeur des valeurs mesurées d'une direction axiale de la pluralité de directions axiales sur une valeur des valeurs mesurées d'une autre direction axiale de la pluralité de directions axiales pour une première section dans laquelle le sujet balance l'une des jambes pour chacune de premières sections d'enjambée qui correspond à un pas du sujet dans la première période sur la base des premières données chronologiques acquises ;

l'acquisition de deuxièmes données chronologiques qui incluent des deuxièmes valeurs mesurées concernant le déplacement des jambes du sujet dans une deuxième période différente de la première période dans laquelle le sujet se déplace en marchant pour chacune de la pluralité de directions axiales ;

l'acquisition d'une deuxième valeur d'indice qui se rapporte à une valeur des valeurs mesurées d'une direction axiale de la pluralité de directions axiales sur une valeur des valeurs mesurées d'une autre direction axiale de la pluralité de directions axiales pour une deuxième section dans laquelle le sujet balance l'une des jambes pour chacune de deuxièmes sections d'enjambée qui correspond à un pas du sujet dans la deuxième période sur la base des deuxièmes données chronologiques acquises ; et le fait de déterminer si chacune des deuxièmes sections d'enjambée dans la deuxième période correspond ou non à la manière de marcher, sur la base de la première valeur d'indice acquise et de la deuxième valeur d'indice acquise, dans lequel, lorsqu'un pourcentage de la deuxième valeur d'indice pour l'une quelconque des deuxièmes sections d'enjambée par rapport à la première valeur d'indice est inférieur ou égal à une valeur seuil prédéterminée, il est déterminé que la quelconque des deuxièmes sections d'enjambée correspond à la manière de marcher.

8. Dispositif de traitement d'informations comprenant : une unité de commande configurée pour :

acquérir des premières données chronologiques qui incluent des valeurs mesurées concernant un déplacement de jambes d'un sujet dans une première période dans laquelle le sujet se déplace en marchant pour chacune d'une pluralité de directions axiales, acquérir une première valeur d'indice qui se rapporte à une valeur des valeurs mesurées d'une direction axiale de la pluralité de directions axiales sur une valeur des valeurs mesurées d'une autre direction axiale de la pluralité de directions axiales pour une première section dans laquelle le sujet balance l'une des jambes pour chacune de premières sections d'enjambée qui correspond à un pas du sujet dans la première période sur la base des premières données chronologiques acquises, acquérir des deuxièmes données chronologiques qui incluent des deuxièmes valeurs mesurées concernant le déplacement des jambes du sujet dans une deuxième période différente de la première période dans laquelle le sujet se déplace en marchant pour chacune de la pluralité de directions axiales, acquérir une deuxième valeur d'indice qui se rapporte à une valeur des valeurs mesurées d'une direction axiale de la pluralité de directions axiales sur une valeur des valeurs mesurées d'une autre direction axiale de la pluralité de directions axiales pour une deuxième section dans laquelle le sujet balance l'une des jambes pour chacune de deuxièmes sections d'enjambée qui correspond à un pas du sujet dans la deuxième période sur la base des deuxièmes données chronologiques acquises, et déterminer si chacune des deuxièmes sections d'enjambée dans la deuxième période correspond ou non à la manière de marcher, sur la base de la première valeur d'indice acquise et de la deuxième valeur d'indice acquise, dans lequel, lorsqu'un pourcentage de la deuxième valeur d'indice pour l'une quelconque des deuxièmes sections d'enjambée par rapport à la première valeur d'indice est inférieur ou égal à une valeur seuil prédéterminée, il est déterminé que la quelconque des deuxièmes sections d'enjambée correspond à la manière de marcher.

# FIG. 1

FIRST TIME-SERIES DATA IN FIRST PERIOD
(PREDETERMINED WALKING MANNER)

RightLegGyro

FIRST STRIDE SECTION    110

SWING

FIRST SECTION    111    15:57

UP-DOWN
DIRECTION z

LEFT-RIGHT
DIRECTION y

ex) FIRST INDEX
VALUE OF y/x

FRONT-BACK
DIRECTION x

SECOND TIME-SERIES DATA IN SECOND PERIOD

RightLegGyro

SECOND STRIDE SECTION    120

SWING

SECOND SECTION    121    15:57

UP-DOWN
DIRECTION z

LEFT-RIGHT
DIRECTION y

ex) SECOND
INDEX VALUE
OF y/x

FRONT-BACK
DIRECTION x

COMPARISON

# FIG. 2

# FIG. 3

# FIG. 4

201

401 CPU

402 MEMORY

400

403 NETWORK I/F

404 TOUCH PANEL

405 SENSOR DEVICE

210 NETWORK

EP 4 173 565 B1

# FIG. 5

# FIG. 6

INFORMATION MEASURING DEVICE — 201

INFORMATION PROCESSING DEVICE — 100

WALKING SECTION DETECTION UNIT — 601

BASE CALCULATION UNIT — 602

INDEX CALCULATION UNIT — 603

DECISION VALUE CALCULATION UNIT — 604

SECTION EXTRACTION UNIT — 605

DATA DISPLAY UNIT — 606

WALKING MEASUREMENT DATA DB — 611

INDEX VALUE DATA DB — 612

EXTRACTED SECTION DATA DB — 613

EP 4 173 565 B1

# FIG. 7

CLASSIFICATION OF SECTIONS

4. MIDDLE LEG SWING PHASE

711

5. DECELERATION PHASE

LEFT LEG

3. ACCELERATION PHASE

708

6. HEEL STRIKE

8. MIDDLE STANCE PHASE

GYRO SENSOR VALUE = 0

709 710

RIGHT LEG

2. TOE OFF

704 705 706 707

702 703

701

[LIST OF ANALYSIS TARGET SECTIONS]

701 STRIDE SECTION
702 SWING SECTION
703 TWO-LEG SUPPORT SECTION
704 FIRST HALF SWING SECTION
705 SECOND HALF SWING SECTION
706 FIRST HALF TWO-LEG SUPPORT SECTION
707 SECOND HALF TWO-LEG SUPPORT SECTION
708 FORWARD SWING SECTION
709 FIRST HALF FORWARD SWING SECTION
710 SECOND HALF FORWARD SWING SECTION
711 STANCE SECTION

[LIST OF WALKING EVENTS (FEATURE POINTS)]
✕ HEEL-STRIKE POINT   △ TOE-OFF POINT
○ FASTEST SWING POINT
□ TWO-LEG SUPPORT MIDPOINT
▲ FORWARD SWING START POINT
   (ACCELERATION PHASE)
   (POINT OF GYRO SENSOR VALUE = 0)
✕ FORWARD SWING END POINT
   (DECELERATION PHASE)
   (POINT OF GYRO SENSOR VALUE = 0)

EP 4 173 565 B1

# FIG. 8

WALKING MEASUREMENT DATA OF WALKING TEST

STRIDE SECTION ~810

FORWARD SWING

FORWARD SWING SECTION          15:57

EP 4 173 565 B1

# FIG. 9

STRIDE SECTION — 810

FORWARD SWING

FORWARD SWING SECTION        15:57

SENSOR VALUES FOR EACH SECTION — 900

| GYRO SENSOR VALUE OF FORWARD SWING SECTION | | | GYRO SENSOR VALUE OF RESIDUAL SECTION | | |
|---|---|---|---|---|---|
| x | y | z | x | y | z |
| 14.61656 | -12.7791 | -94.1791 | -109.413 | 19.2394 | 22.38132 |
| 73.1182 | -12.9757 | -61.9903 | -124.88 | 20.69195 | 47.19959 |
| 117.1153 | -13.5567 | -16.6428 | -145.183 | 19.29931 | 62.69902 |
| 159.5555 | -15.3994 | -22.3049 | -167.48 | 16.58274 | 87.00175 |
| 205.9797 | -19.3551 | -52.8382 | -183.777 | 13.59008 | 120.5497 |
| 244.3217 | -18.2961 | -78.6192 | -202.096 | 18.43173 | 146.6369 |
| 273.3878 | -13.0305 | -90.9076 | -221.389 | 32.31421 | 116.3295 |
| 297.4108 | -8.23012 | -102.888 | -206.482 | 34.35389 | 56.41056 |
| 316.5019 | -3.15083 | -110.145 | -152.941 | 15.19254 | 7.29661 |
| 335.9532 | 2.207595 | -123.405 | -96.9245 | 5.271198 | -37.2864 |
| 356.1744 | 7.256999 | -145.786 | -58.3237 | -7.73703 | -2.52388 |
| ... | ... | ... | ... | ... | ... |

EP 4 173 565 B1

# FIG. 10

AVERAGE VALUES FOR EACH SECTION 1000

| AVERAGE VALUES OF GYRO SENSOR VALUES IN x-, y-, AND z-AXES | | | |
|---|---|---|---|
| SECTION | x | y | z |
| FORWARD SWING | 247.519 | -11.728 | -50.1079 |
| RESIDUE | -119.345 | 4.10149 | 19.03216 |

⇨ CALCULATE AVERAGE VALUE FOR EACH AXIS (x, y, AND z)

$$\text{mean} = \frac{1}{n} \sum_{k=1}^{n} a_k$$

n: NUMBER OF GYRO SENSOR VALUES
$a_k$: k-TH GYRO SENSOR VALUE

INDEX VALUES FOR EACH SECTION 1010

| INDEX VALUE OF EACH SECTION | | | |
|---|---|---|---|
| SECTION | x | y | z |
| FORWARD SWING | 1 | -0.04738 | -0.20244 |
| RESIDUE | 1 | -0.03437 | -0.15947 |

⇨ CALCULATE RATIO BASED ON x-AXIS

$$\text{Index}_y = \frac{\text{mean}_y}{\text{mean}_x} , \text{Index}_z = \frac{\text{mean}_z}{\text{mean}_x}$$

mean: AVERAGE VALUE OF EACH AXIS

EP 4 173 565 B1

# FIG. 11

EP 4 173 565 B1

CALCULATE AVERAGE VALUE OF
INDEX VALUES OF ALL STRIDE SECTIONS

$$\text{Base}_y = \frac{1}{n}\sum_{k=1}^{n} \text{Index}_{yk}, \quad \text{Base}_z = \frac{1}{n}\sum_{k=1}^{n} \text{Index}_{zk},$$

n: NUMBER OF STRIDE SECTIONS
$\text{Index}_{yk}$: INDEX VALUE OF k-TH STRIDE SECTION
IN y-AXIS DIRECTION
$\text{Index}_{zk}$: INDEX VALUE OF k-TH STRIDE SECTION
IN z-AXIS DIRECTION

⇒

1100

BASE INDEX VALUES CALCULATED FROM
WALKING TEST DATA

| BASE INDEX VALUE | | | |
|---|---|---|---|
| SECTION | x | y | z |
| FORWARD SWING | 1 | -0.0481 | -0.1564 |
| RESIDUE | 1 | -0.027 | -0.1823 |

# FIG. 12

WALKING MEASUREMENT DATA OF DAILY WALKING

1200

EP 4 173 565 B1

# FIG. 13

STRIDE SECTION 1210

FORWARD SWING

FORWARD SWING SECTION    15:57

⟹

## SENSOR VALUES FOR EACH SECTION    1300

| GYRO SENSOR VALUE OF FORWARD SWING SECTION | | | GYRO SENSOR VALUE OF RESIDUAL SECTION | | |
|---|---|---|---|---|---|
| x | y | z | x | y | z |
| 14.61656 | -12.7791 | -94.1791 | -109.413 | 19.2394 | 22.38132 |
| 73.1182 | -12.9757 | -61.9903 | -124.88 | 20.69195 | 47.19959 |
| 117.1153 | -13.5567 | -16.6428 | -145.183 | 19.29931 | 62.69902 |
| 159.5555 | -15.3994 | -22.3049 | -167.48 | 16.58274 | 87.00175 |
| 205.9797 | -19.3551 | -52.8382 | -183.777 | 13.59008 | 120.5497 |
| 244.3217 | -18.2961 | -78.6192 | -202.096 | 18.43173 | 146.6369 |
| 273.3878 | -13.0305 | -90.9076 | -221.389 | 32.31421 | 116.3295 |
| 297.4108 | -8.23012 | -102.888 | -206.482 | 34.35389 | 56.41056 |
| 316.5019 | -3.15083 | -110.145 | -152.941 | 15.19254 | 7.29661 |
| 335.9532 | 2.207595 | -123.405 | -96.9245 | 5.271198 | -37.2864 |
| 356.1744 | 7.256999 | -145.786 | -58.3237 | -7.73703 | -2.52388 |
| ... | ... | ... | ... | ... | ... |

⟹

EP 4 173 565 B1

# FIG. 14

1400

AVERAGE VALUES FOR EACH SECTION

| AVERAGE VALUES OF GYRO SENSOR VALUES IN x-, y-, AND z-AXES | | | |
|---|---|---|---|
| SECTION | x | y | z |
| FORWARD SWING | 247.519 | -11.728 | -50.1079 |
| RESIDUE | 119.345 | 4.10149 | 19.03216 |

CALCULATE AVERAGE VALUE
FOR EACH AXIS (x, y, AND z)

$$mean = \frac{1}{n}\sum_{k=1}^{n} a_k$$

n: NUMBER OF PIECES OF DATA OF EACH SECTION
$a_k$: k-TH SENSOR VALUE

1410

INDEX VALUES FOR EACH SECTION

| INDEX VALUE OF EACH SECTION | | | |
|---|---|---|---|
| SECTION | x | y | z |
| FORWARD SWING | 1 | -0.04738 | -0.20244 |
| RESIDUE | 1 | -0.03437 | -0.15947 |

CALCULATE RATIO BASED ON x-AXIS

$$Index_y = \frac{mean_y}{mean_x}, Index_z = \frac{mean_z}{mean_x}$$

mean: AVERAGE VALUE OF EACH AXIS

CALCULATE IN ALL STRIDE SECTIONS

# FIG. 15

~1200

WALKING MEASUREMENT DATA OF DAILY WALKING

LeftLegGyro

RightLegGyro

16:12:29 16:12:32 16:12:35 16:12:38 16:12:41 16:12:44 16:12:47 16:12:50 16:12:53

1410

INDEX VALUE OF ONE STRIDE SECTION

**INDEX VALUE OF ONE STRIDE SECTION**

| SECTION | x | y | z |
|---|---|---|---|
| FORWARD SWING | 1 | -0.08068 | -0.08336 |
| RESIDUE | 1 | 0.022162 | -0.27155 |

CALCULATE RATIOS OF BASE INDEX VALUE OF WALKING TEST AND INDEX VALUES OF y- AND z-AXES
SPECIFY AVERAGE VALUE OF RATIOS AS DECISION VALUE

1500

**RATIO OF INDEX VALUE**

| SECTION | x | y | z | DECISION VALUE |
|---|---|---|---|---|
|  |  |  |  | AVERAGE |
| FORWARD SWING | - | 68% | 47% | 45% |
| RESIDUE | - | 18% | 49% |  |

CALCULATE DECISION VALUE FOR WHOLE PLURALITY OF STRIDE SECTIONS

CALCULATE RATIO OF BASE INDEX VALUE AND INDEX VALUE OF ONE STRIDE SECTION

$$\text{Ratio}_y = \left| 1 - \left| \frac{\text{compIndex}_y}{\text{baseIndex}_y} \right| \right|, \text{Ratio}_z = \left| 1 - \left| \frac{\text{compIndex}_z}{\text{baseIndex}_z} \right| \right|$$

baseIndex: BASE INDEX OF WALKING TEST, compIndex: INDEX VALUE OF ONE STRIDE SECTION OF DAILY WALKING

1100

BASE INDEX VALUES CALCULATED BASED ON WALKING MEASUREMENT DATA OF WALKING TEST

**BASE INDEX VALUE**

| SECTION | x | y | z |
|---|---|---|---|
| FORWARD SWING | 1 | -0.0481 | -0.1564 |
| RESIDUE | 1 | -0.027 | -0.1823 |

# FIG. 16

1200

WALKING MEASUREMENT DATA OF DAILY WALKING
(INCLUDING WALKING OTHER THAN NORMAL WALKING)

DECISION VALUE
OF EACH STRIDE   45%,40%,30%,31% ···,        44%,58%,78%,84%,51%,42%,35%,38% ···,
SECTION

$Result_i = Threshold \geq decision_i$
Threshold: THRESHOLD VALUE, $decision_i$: DECISION VALUE OF i-TH STRIDE SECTION

NORMAL WALKING SECTION
EXTRACTION RESULT        1601            1602            1601      1200
(VISUALIZATION IMAGE)

EP 4 173 565 B1

# FIG. 17

START

ACQUIRE FIRST TIME-SERIES DATA — S1701

IDENTIFY PLURALITY OF STRIDE SECTIONS INCLUDED IN FIRST PERIOD — S1702

SELECT ANY ONE OF STRIDE SECTIONS — S1703

DIVIDE SELECTED STRIDE SECTION INTO FORWARD SWING SECTION AND RESIDUAL SECTION — S1704

CALCULATE AVERAGE VALUE OF GYRO SENSOR VALUES — S1705

CALCULATE INDEX VALUE — S1706

HAVE ALL STRIDE SECTIONS BEEN SELECTED? — S1707
NO
YES

CALCULATE AVERAGE VALUE OF INDEX VALUES — S1708

SET BASE AVERAGE VALUE — S1709

END

FIG. 18

```
                    ( START )
                        │
                        ▼               S1801
        ┌───────────────────────────────────┐
        │    ACQUIRE SECOND TIME-SERIES DATA │
        └───────────────────────────────────┘
                        │               S1802
                        ▼
        ┌───────────────────────────────────┐
        │  IDENTIFY PLURALITY OF STRIDE      │
        │  SECTIONS INCLUDED IN SECOND PERIOD│
        └───────────────────────────────────┘
                        │               S1803
                        ▼
        ┌───────────────────────────────────┐
        │    SELECT ANY ONE OF STRIDE SECTIONS│
        └───────────────────────────────────┘
                        │               S1804
                        ▼
        ┌───────────────────────────────────┐
        │ DIVIDE SELECTED STRIDE SECTION INTO│
        │ FORWARD SWING SECTION AND          │
        │ RESIDUAL SECTION                   │
        └───────────────────────────────────┘
                        │               S1805
                        ▼
        ┌───────────────────────────────────┐
        │ CALCULATE AVERAGE VALUE OF GYRO    │
        │ SENSOR VALUES                      │
        └───────────────────────────────────┘
                        │               S1806
                        ▼
        ┌───────────────────────────────────┐
        │       CALCULATE INDEX VALUE        │
        └───────────────────────────────────┘
                        │               S1807
                        ▼
        ┌───────────────────────────────────┐
        │     ACQUIRE BASE AVERAGE VALUE     │
        └───────────────────────────────────┘
                        │               S1808
                        ▼
        ┌───────────────────────────────────┐
        │      CALCULATE DECISION VALUE      │
        └───────────────────────────────────┘
                        │
                        ▼               S1809
                    IS DECISION                  NO
            VALUE EQUAL TO OR LOWER THAN ─────────┐
                 THRESHOLD VALUE?                 │
                        │ YES                     │
                        ▼               S1810     │
        ┌───────────────────────────────────┐    │
        │    EXTRACT NORMAL WALKING SECTION  │    │
        └───────────────────────────────────┘    │
                        │◄───────────────────────┘
                        ▼               S1811
            NO      HAVE ALL STRIDE
        ┌────── SECTIONS BEEN SELECTED?
        │               │ YES
        │               ▼               S1812
        │   ┌───────────────────────────────────┐
        │   │   STORE NORMAL WALKING SECTION     │
        │   └───────────────────────────────────┘
        │               │
        │               ▼
        │           ( END )
        └──────────────┘
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018042525 A
- US 20170281054
- WO 2020045371 A
- EP 3847961 A1
- WO 2017109920 A